# EUROPEAN PATENT APPLICATION

(11) **EP 3 272 878 A1**
(43) Date of publication of application: **24.01.2018**
(21) Application number: 16180434.9
(22) Date of filing: 20.07.2016
(51) Int. Cl.: C12Q 1/68, A61K 38/48, C12N 15/86, A61K 48/00, G01N 33/68

(54) **METHODS OF DIAGNOSING AND TREATMENT OF ALZHEIMER'S DISEASE**

(71) Applicant: Ecole Polytechnique Fédérale de Lausanne (EPFL), 1015 Lausanne (CH); Fundació Institut de Recerca Biomèdica de Bellvitge, 08907 Hospitalet del Llobregat-Barcelona (ES)
(72) Inventor: SACHEZ MUT, Jose Vicente, 1015 Lausanne (CH); GRÄFF, Johannes, 1015 Lausanne (CH); HEYN, Holger, 08908 Hospitalet de Llobregat - Barcelona (ES); ESTELLER BADOSA, Manel, 08908 Hospitalet de Llobregat - Barcelona (ES)
(74) Representative: Rigamonti, Dorotea

(57) **Abstract**

The present invention describes a method for the diagnosis or prognosis of Alzheimer's disease (AD), comprising the steps of placing a sample isolated from a person suspected of having AD onto a substrate labeling the sample to identify at least one epigenetic marker and/or genetic variation and determining a state of AD, wherein one of said epigenetic markers is PM20D1 promoter DNA methylation and/or one of said genetic variation is in at least one of PM20D1 mQTL-associated SNPs.

## Description

### DESCRIPTION

### Background

Alzheimer's disease (AD) is the most common cause of dementia in western society. Despite intensive research efforts, its precise etiology remains unknown.

AD is believed to be caused by a combination of genetic and non-genetic risk factors, the latter likely being mediated by epigenetic mechanisms (Chouliaras L et al., Epigenetic regulation in the pathophysiology of Alzheimer's disease. Progress in neurobiology 2010, 90:498-510; Sanchez-Mut JV and Graeff J, Epigenetic Alterations in Alzheimer's disease. Frontiers In Behavioral Neuroscience 2015, 9:347). Genetic risk factors are usually interrogated by genome-wide association studies (GWAS) which have identified an important number of loci associated with AD pathology. However, GWAS tend to result in strong statistical associations of large portions of the genome, for which a causal relationship is difficult to establish. In contrast, locus-specific or epigenome-wide association studies (EWAS) identify locus-specific alterations providing mechanistic insights for a particular risk gene, but lack the statistical significance of GWAS (Rakyan VK et al., Epigenome-wide association studies for common human diseases. Nat Rev Genet. 2011, 12:529-41). Combining both approaches, it has recently become possible to identify single nucleotide polymorphisms (SNPs) that correlate with alterations in DNA methylation levels, named methylation quantitative trait loci (mQTLs), the importance of which for complex diseases is just starting to be recognized. As an example, mQTLs have been reported for cancer, osteoarthritis, multiple sclerosis, post-traumatic stress disorder and obsessive-compulsive and bipolar disorders but, thus far, not for neurodegenerative diseases such as AD, where there is still a need of new biomarkers and thus new methods of prognosis to better identify poor prognosis patients in AD.

Peptidase M20 domain containing 1 (PM20D1) is a protein coding gene that has been recurrently identified as a mQTL (IPDGC and WTCCC2, A Two-Stage Meta-Analysis Identifies Several New Loci for Parkinson's Disease. Plos Genet. 2011) and expression quantitative trait loci (eQTL) (Grundberg E et al., Global Analysis of the Impact of Environmental Perturbation on cis-Regulation of Gene Expression. Plos Genet. 2011). mQTL SNPs PM20D1 associated are known from Heyn H et al. 2013 (Heyn H et al., DNA methylation contributes to natural human variation. Genome Res. 2013, 23(9):1363-72). Today, while the ultimate pathology of Alzheimer's disease is fairly well established, effective diagnostic methods and treatment modalities remain in fact elusive because of the complex biological basis for the etiology and pathogenesis of the disease. Clinical diagnostic techniques for Alzheimer's disease currently rely on screening individuals displaying symptoms of dementia by excluding other possible causes such as depression, poor nutrition, other dementing conditions (e.g., Parkinson's disease with dementia), or drug interactions. These qualitative and unspecific methods often leave AD misdiagnosed or unrecognized until later stages in the disease when treatments may be less effective. Genetic testing have been developed so far, useful in defining clinical trial project but not 100% capable to test for pathology at a single individual level (Albertici A et al., Clinical, genetic, and neuroimaging features of Early Onset Alzheimer Disease: the challenges of diagnosis and treatment. Curr. Alzheimer Res. 2014, 11(10):909-17). Imaging techniques are also largely applied in AD testing (Schindler SE et al., Advances in diagnostic testing for Alzheimer disease. Mo Med. 2013, 110 (5) : 401-5) .

There is therefore a need for specific diagnostic tests and biomarkers that are predictive of a subject developing AD and that can help establish a clear prognosis of the disease.

### Description

Various embodiments of the present description provide methods for the detection, the diagnosis, and/or the prediction of disease onset of AD. Further areas of applicability will become apparent from the description provided herein. It should be understood that the description and specific examples are intended for purposes of illustration only and are not intended to limit the scope of the present teachings.

### Figure Description

The drawing figures described herein are for illustration purposes only and are not intended to limit the scope of the present teachings in any way. The present teachings will become more fully understood from the detailed description and the accompanying drawing figures wherein:

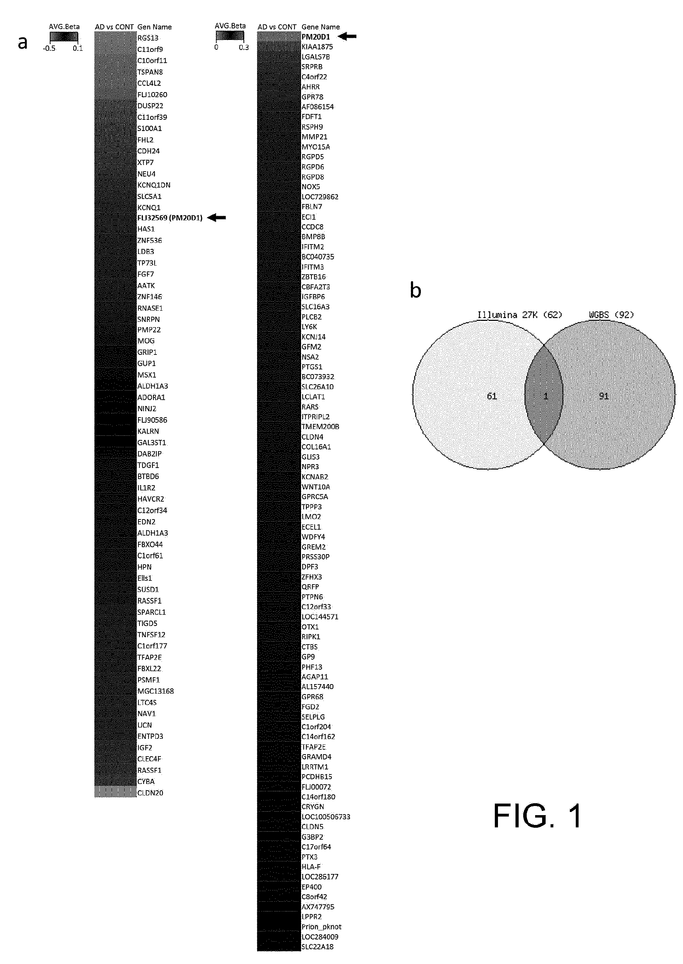

Figure 1: a) Heatmap of differentially methylated genes identified in the GSE45775 Illumina 27K DNA methylation arrays and hippocampus samples of AD (left), and GSE57361 Whole genome Bisulphite sequencing (WGBS) and frontal cortex samples of AD (right); b) Venn diagram showing that only PM20D1 is commonly hyper methylated in both studies.

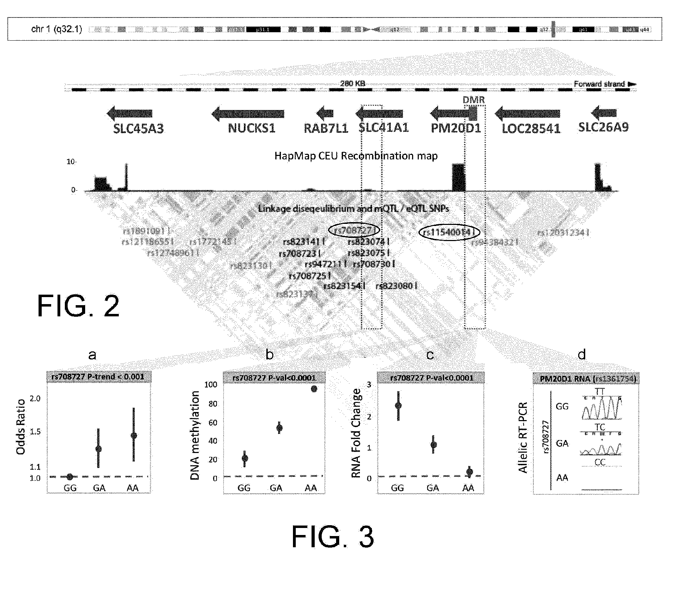

Figure 2: overview of the rs708727-PM20D1 locus. The region comprises several genes, indicated by arrows. Below the genes, the rate of recombination peaks in the gene body of PM20D1 dividing rs708727 and the PM20D1 promoter in two independent linkage-disequilibrium blocks, according to the HapMap CEU recombination map indicated below. rs708727 (circled), located in exon six of the neighbouring gene SLC41A1, is in linkage-disequilibrium with other SNPs of the block, among which rs708730, located on the same SCL41A1 gene. rs11540014 (circled), located in the promoter of PM20D1, is in linkage disequilibrium with other SNPs of its own block. The differentially methylated region (DMR) on PM20D1 promoter is highlighted in black.
Figure 3: a) rs708727 shows an allele-dose dependent association with AD; b) rs708727 correlates with the levels of PM20D1 DNA methylation in human brain samples measured by pyrosequencing; c) rs708727 correlates with PM20D1 expression in human brain samples measured by quantitative Real-Time PCR; d) rs1361754 is located in the coding region of PM20D1 and it is in linkage disequilibrium with rs708727. Retro transcription PCR Sanger sequencing of rs1361754 heterozygous samples detects rs1361754 (T) but not rs1361754 (A) RNA transcripts, indicating that only the rs708727 (G) chromatid is active.

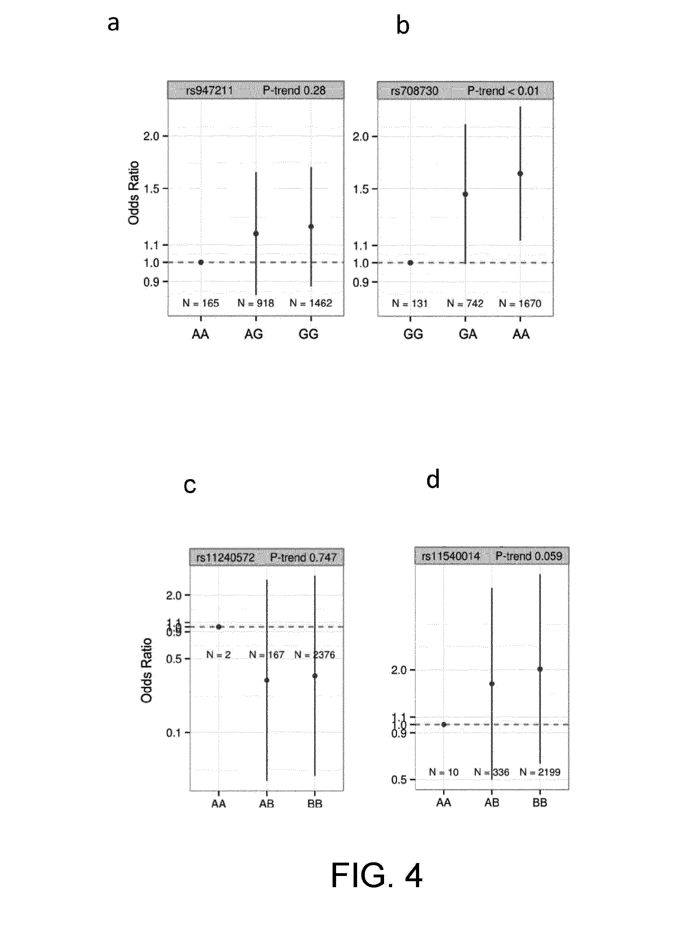

Figure 4: AD association analysis of SNPs located in both linkage-disequilibrium blocks of rs708727-PM20D1 promoter region. a), b) mQTL SNPs located in the rs708727 linkage-disequilibrium region are also associated with AD. c), d) SNPs located in PM20D1 promoter linkage-disequilibrium region are not associated with AD.

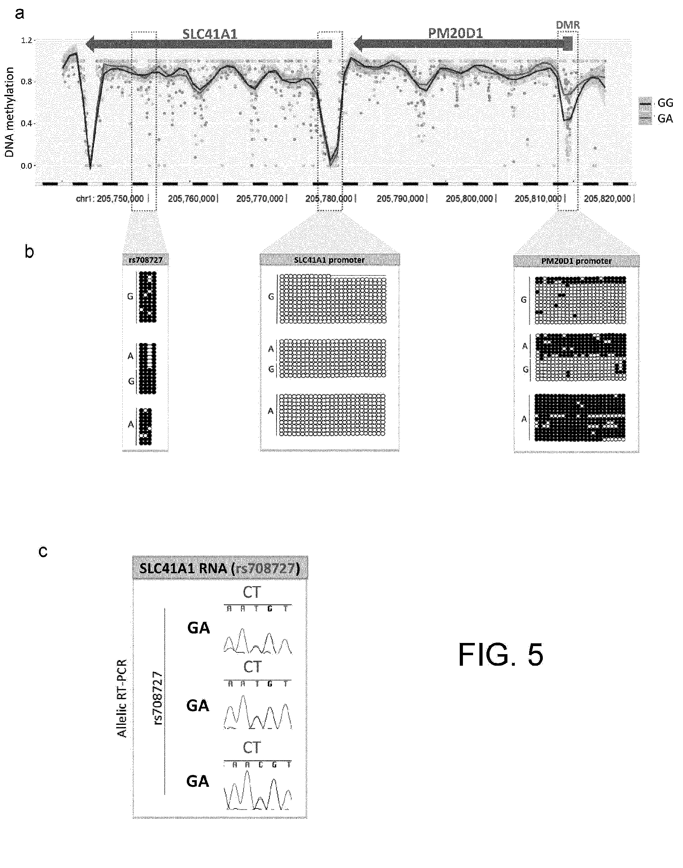

Figure 5: a) whole genome bisulfite sequencing analysis of DNA methylation in GG and GA rs708727 human frontal cortex samples. Differences in DNA methylation are restricted to the DMR detected in PM20D1 promoter; b) locus-specific bisulfite sequencing of the rs708727, SLC41A1 and PM20D1 promoter regions confirming the whole genome bisulfite sequencing and pyrosequencing data in GG, GA and AA rs707827 human frontal cortex samples; c) SLC41A1 expression from three different heterozygous samples, results obtained by RT-PCR Sanger sequencing.

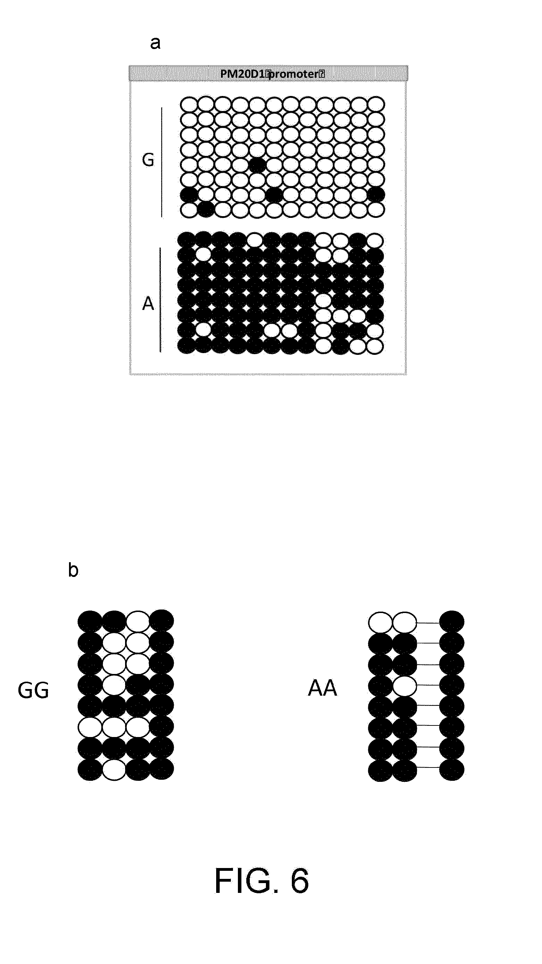

Figure 6: a) PM20D1 promoter DNA methylation is correlated with rs708727 genotypes in immortalized B cells measured by bisulfite sequencing/cloning; b) rs708727 region DNA methylation is not correlated with rs708727 genotypes in immortalized B cells measured by bisulfite sequencing/cloning.

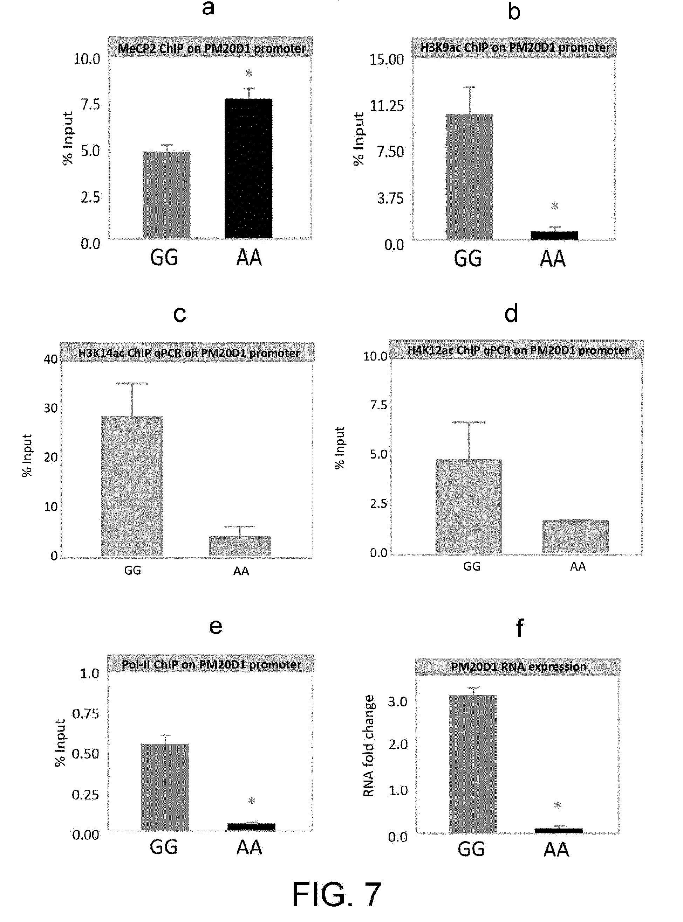

Figure 7: a) Chromatin immunoprecipitation assay (ChIP) showing higher binding of MeCP2 in rs708727 AA methylated samples on the PM20D1 promoter region; b) ChIP showing that PM20D1 promoter region of rs708727 AA samples is depleted of H3K9ac; c) ChIP showing that PM20D1 promoter region of rs708727 AA samples is depleted of H3K14ac; d) ChIP showing that PM20D1 promoter region of rs708727 AA samples is depleted of H4K12ac; e) ChIP showing lower binding of Polymerase-II in the PM20D1 promoter; f) qRT-PCR showing lower PM20D1 RNA levels in rs708727 AA samples.

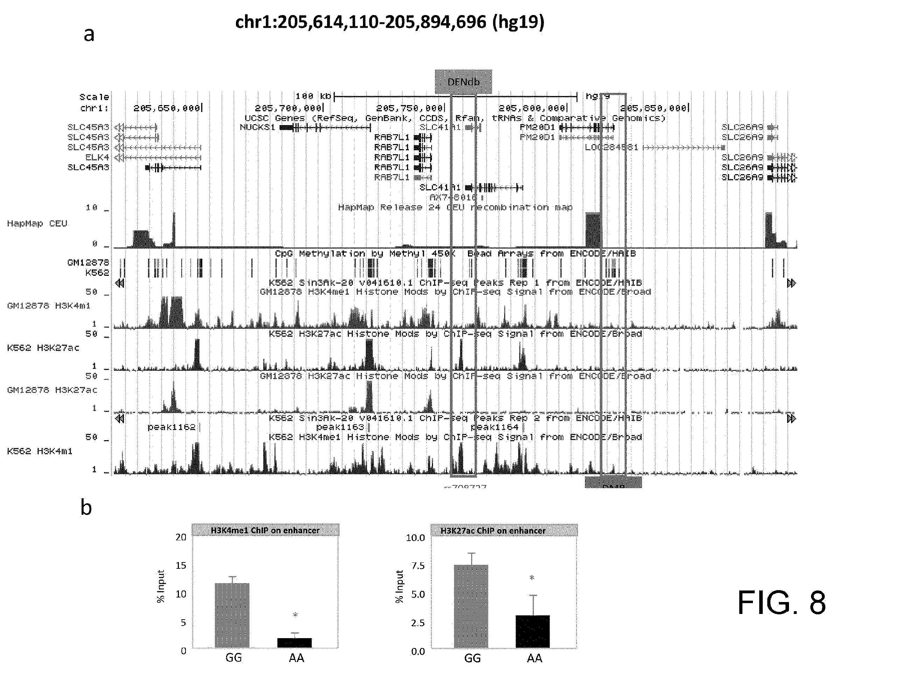

Figure 8: a) enhancer-enriched epigenetic marks such as histone 3 lysine 4 mono-methylation (H3K4me1) and H3K27ac in cells lines for which the DNA methylation profile was also available using ENCODE (Encyclopedia of DNA Elements; ENCODE/Broad Histone Track for H3K4me1 and H3K27ac, and HAIB Methyl450 Track for K562 and GS12878 cell lines) showing a peak of H3K4me1 and H3K27ac at 10kb downstream of rs708727 in the PM20D1 promoter non-methylated K562 cells, suggesting the existence of allele-dependent active enhancer. b) ChIP assay showing that H3K4me1 (left) and H3K27ac (right) are enriched in rs708727 (GG) samples in the rs708727-enhancer locus.

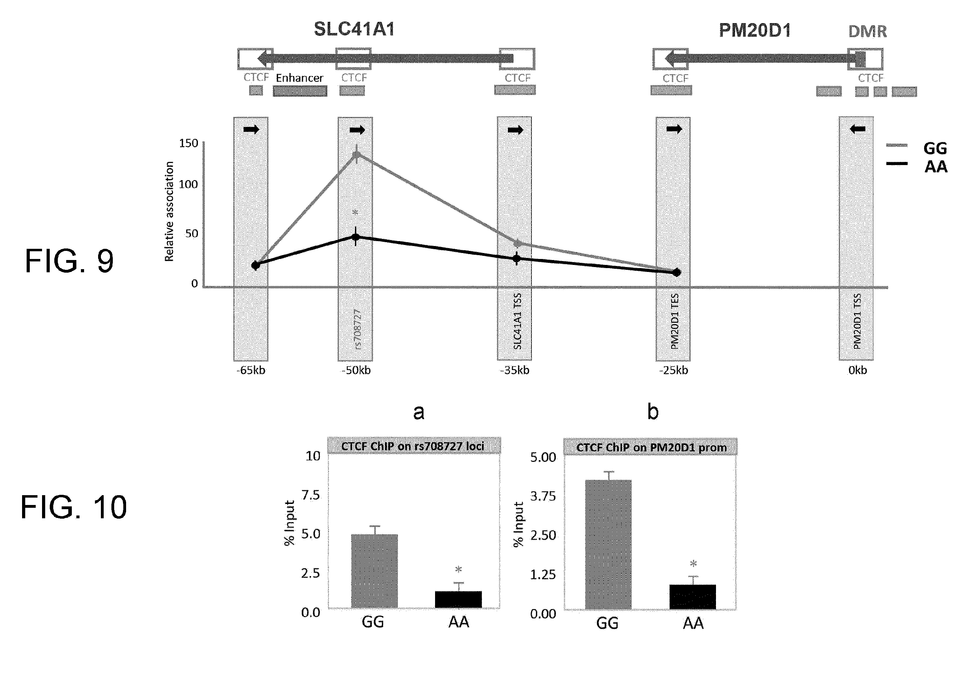

Figure 9: relative interaction frequencies between regions as a function of the genomic distances (in Kb) and rs708727 genotype.
Figure 10: ChIP assay showing the binding of CTCF on rs708727 loci (a) and on PM20D1 promoter region (b).

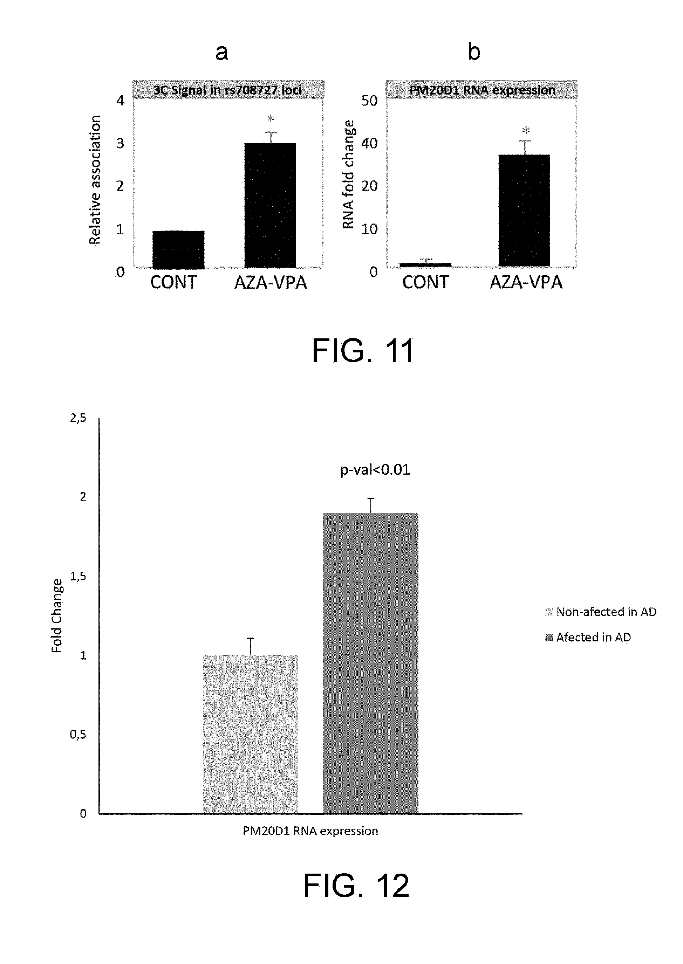

Figure 11: a combination of 5-aza/VPA restores 3C DNA (a) and PM20D1 expression levels (b).
Figure 12: PM20D1 RNA expression in AD-affected and non-affected mouse brain areas.

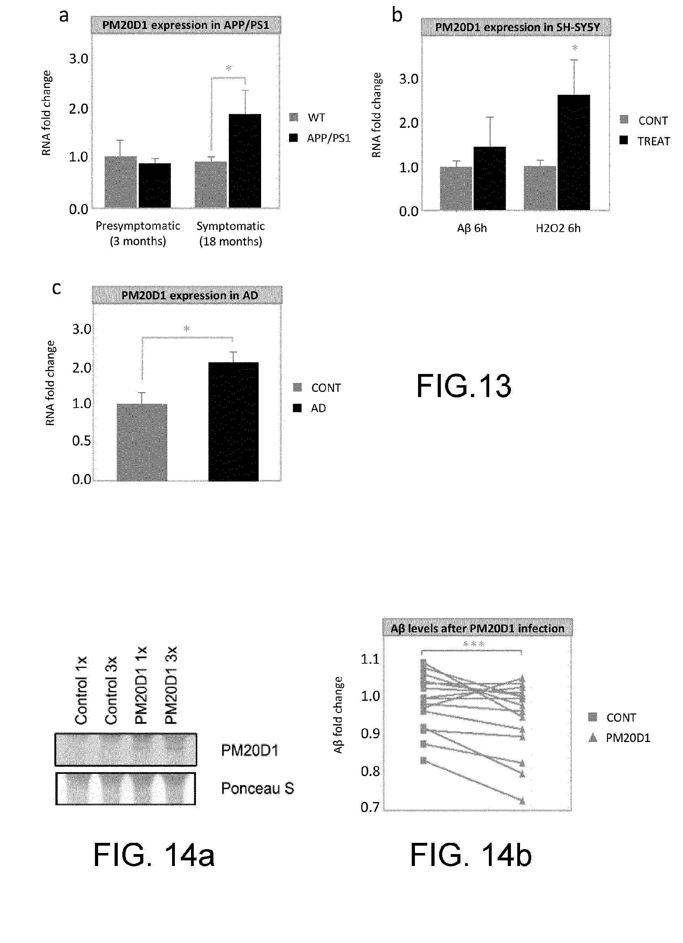

Figure 13: PM20D1 RNA expression a) in wt and APP/PS1 mice; b) in neuroblastoma cells, following exposure to AD-related stressors such as reactive oxygen species produced by H2O2 and Aβ; c) in rs708727 genotype-corrected frontal cortex of human AD samples. Data are presented as means plus standard error. *p<0.05 by student's t-test.
Figure 14: a) overexpressed PM20D1 in hippocampal primary culture is detected in culture media; b) PM20D1 transduction decreases Ab levels in hippocampal primary cultures. Data represent 15 different paired experiments performed (mock versus PM20D1 transduction) with three different virus batch productions (five experiments per virus batch). ***p<0.001; paired t-test.

### Correlation observed in human samples

The inventors analyzed the epigenetic profile (methylation profile of the histones) in an enlarged cohort of AD patients. Hippocampus and frontal cortex of AD samples have been analyzed. EWAS studies surprisingly identified only a single DNA hyper methylation common to hippocampus and frontal cortex samples analyzed, consisting in a hyper methylation of PM20D1, as reported in Figure 1.

Following from this observation, previously associated PM20D1 mQTL SNPs (Heyn et al. 2013, previously cited), reported in Figure 2, have been selected to evaluate their relation, if any, with AD. Data from a public available database from NCBI GWAS studies (phs000168.v1.p1 NIA) have been examined by fitting a Bayesian univariate logistic regression with phenotype as outcome. To note, the analyzed data were obtained in blood sample. A significant allele-dose dependent association of SNP rs708727 with the odds ratio for developing AD has been detected (P-trend <0.001, Figure 3a). Supporting this observation, four other reported PM20D1 mQTL and eQTL SNPs, located in the linkage-disequilibrium region of rs708727, rs947211, rs708730, rs708724 and rs823130, also correlated with AD (Figure 4a and b; Table 1, bold), albeit not as strongly.

**Table 1: AD association analysis of eQTLs from GTEX Consortium 2015, Science, included in the phs000168.v1.p1 NIA - Late Onset AD and National Cell Repository for AD Family Study. Major and minor alleles and linkage disequilibrium data from SNAP Broad Institute database are also indicated.**

| **SNP** | **Distance** | **RSquared** | **DPrime** | **Major** | **Minor** | **LD Block** | **eQTL Effect Size** | **eQTL P-Value** | **AD p.value** | **AD model** |
|---|---|---|---|---|---|---|---|---|---|---|
| **rs823130** | 53513 | 0,745 | 0,892 | T | C | SNP | -0,8 | 5,60E-35 | 0,02738 | additive |
| **rs708724** | 24222 | 0,838 | 0,931 | C | A | SNP | -0,78 | 1,80E-32 | 0,01599 | additive |
| **rs947211** | 15220 | 0,258 | 1 | G | A | SNP | -0,42 | 1,6E-06 | 0,28850 | additive |
| **rs708727** | 0 | 1 | 1 | A | G | SNP | -0,92 | 1,70E-49 | 0,00076 | additive |
| **rs708730** | 9895 | 0,137 | 1 | A | G | SNP | -0,45 | 6,2E-06 | 0,00990 | additive |
| rs1361754 | 33987 | 0,666 | 1 | G | A | SNP | -0,77 | 5,40E-25 | - | - |
| rs11240572 | 11091 | 0,002 | 1 | C | A | Recomb | - | - | 0,75510 | additive |
| rs11540014 | 0 | 1 | 1 | T | C | site promoter | - | - | 0,05884 | additive |
| rs12031234 | 59584 | 0,908 | 1 | T | G | promoter | - | - | 0,07426 | additive |

Contrarily, SNPs located in the PM20D1 promoter linkage-disequilibrium region were not associated with AD (Figure 4c and d; underlined in Table 1). Remarkably, rs708727, rs947211, rs708730, rs708724 and rs823130, and the PM20D1 promoter region are located in two independent linkage disequilibrium regions (HapMap Figure 2), therefore excluding the mQTL/eQTL association to be the mere consequence of a linkage-disequilibrium effect.

By testing frontal cortex sample, a strong correlation in a rs708727 haplotype and dose-dependent manner with PM20D1 DNA methylation has been observed (Figure 3b, P-val<0.0001; Pearson correlation) but also with PM20D1 RNA levels (Figure 3c, P-val<0.0001; Pearson correlation). Similar associations have been observed in different tissues at RNA levels (data not shown) suggesting that the correlation is independent from the cell origin. Furthermore, PM20D1 is monoallelically expressed and it is only detectable from rs708727 G chromatids in heterozygous samples (Figure 3d). As a control, SLC41A1 expression has been evaluated, since rs708727 lies in the coding region of SLC41A1 (Figure 2). SLC41A1 does not show differences in DNA methylation and its RNA can be detected from both chromatids (Figure 5a, b, c). Taken together, these findings surprisingly indicate that PM20D1 methylation and expression are dependent on the rs708727 SNP, and that both, the epigenetic regulation of PM20D1 and the genetic variation at rs708727, are linked to AD.

### Assays on immortalized B cells

B cells have been immortalized from rs708727 GG and AA carriers. Similar to the frontal cortex samples, rs708727 genotype was associated with DNA methylation levels of PM20D1 in a haplotype dose-dependent manner (Figure 6a), while the DNA methylation in the rs708727 region did not differ between genotypes (Figure 6b). *Methyl CpG Binding Protein 2 (MeCP2)* binding has been then evaluated and higher binding levels were observed in rs708727 AA carriers (Figure 7a), wherein rs708727 AA carriers showed lower levels of histone 3 lysine 9 acetylation (H3K9ac) (Figure 7b) and of H3K14ac and H4K12ac (Figure 7c and d), which were accompanied by lower RNA polymerase-II accessibility (Figure 7e) and reduced PM20D1 RNA expression (Figure 7f), in agreement with the known interaction of MECP2 with histone deacetylase 1 (HDAC1; Suzuki et al. Direct association between PU.1 and MeCP2 that recruits mSin3A-HDAC complex for PU.1-mediated transcriptional repression. Oncogene 2003, 22).

The genetic region of chromosome 1 encompassing rs708727 and PM20D1 has been then screened for enhancer-enriched epigenetic marks such as histone 3 lysine 4 mono-methylation (H3K4me1) and H3K27ac in cells lines for which the DNA methylation profile was also available using ENCODE (Encyclopedia of DNA Elements; ENCODE/Broad Histone Track for H3K4me1 and H3K27ac, and HAIB Methyl450 Track for K562 and GS12878 cell lines). A peak of H3K4me1 and H3K27ac has been detected at 10kb downstream of rs708727 in the PM20D1 promoter non-methylated K562 cells, which posits the existence of allele-dependent active enhancer (Figure 8a). Supporting this observation, an enrichment of H3K4me1 and H3K27ac has been observed in rs708727 GG samples (Figure 8b). Considering that enhancers often physically interact with their target promoters (Whalen S et al. Enhancer-promoter interactions are encoded by complex genomic signatures on looping chromatin. Nature Genetics 2016, 48:488-496), a chromatin conformation capture assay (3C) has been performed in rs708727 GG (square) and AA (circle) carrier B cells. PM20D1 promoter region has been found to interact with rs708727 region, but also this interaction was rs708727 genotype-dependent and weakened in the PM20D1 transcriptionally silent genotype (AA) (Figure 9). In line, CCCTC-binding factor (CTFC), which is responsible for more than 90% of DNA loops in mammalian genomes and negatively affected by DNA methylation, showed reduced binding to the rs708727 and PM20D1 regions in AA carriers (Figure 10a, b).

B cells were then treated with the DNA demethylating agent 5-Azacytidine-dC (5-aza) in combination with the histone deacetylase inhibitor valproic acid (VPA). 5-aza/VPA treatment restored CTFC binding and allowed for the rs708727-PM20D1 chromatin loop to reform (Figure 11a). Moreover, the treatment increased PM20D1 RNA expression (Figure 11b).

The reported experiments clearly demonstrate a genotype-dependent three-dimensional chromatin interaction between the rs708727 region, with enhancer-like characteristics, and the promoter region of PM20D1, which display a genotype and loop-dependent differential expression.

### Correlation observed in AD mouse model

The extensively used APP/PS1 mouse model of AD has been used. According to Allen Brain Atlas mouse database, AD affected and non-affected brain area have been separated and tested for PM20D1 RNA expression levels. The obtained data are reported in Figure 12, indicating an higher expression in AD affected brain regions.

Frontal cortex has then been analyzed in wt and APP/PS1 mice, at 3 and 18 months. At 3 months, the APP/PS1 mice are presymptomatic and no differences in PM20D1 expression are observed. At 18 months, the APP/PS1 mice are symptomatic and a significative difference is observed, where PM20D1 expression is increased in AD (Figure 13a).

### Assay in SH-SY5Y neuroblastoma cells

SH-SY5Y cells were exposed to stimuli extensively used to mimic AD intracerebral insult, i.e. oxidative stress and Amyloid-β. Cells were treated with 0.02% H₂O₂ or with 50µM Aβ during 6 hours. Before and after treatment, PM20D1 expression levels were evaluated. The results, reported in Figure 13b, indicate a stimuli-induced increase in PM20D1 expression.

### PM20D1 expression in human samples

PM20D1 RNA expression was analyzed in a publicly available database from NCBI expression studies (GSE330000). The represented AD-samples showed lower levels of PM20D1 with respect to control samples (log.Fold change -0.0195; P-val<0.05). This data confirm the correlation AA rs708727 and AD, wherein PM20D1 is monoallelically expressed and it is only detectable from rs708727 G chromatids in heterozygous samples (Figure 3d). The collected and genotyped frontal cortex samples were then analysed. After genotype correction, an increase of PM20D1 expression was observed in AD samples, in line with the observation in in vitro and AD mouse model (Figure 13c) and demonstrating that G rs708727 carriers upregulate PM20D1 in AD.

### Aβ levels following PM20D1 overexpression

Primary hippocampal cultures obtained from P0 APPswe/PS1dE9 mice (Borchelt et al. 1997) were cultured in media consisting of Neurobasal (Invitrogen), B27 supplement (Invitrogen), L-glutamine (Invitrogen) and penicillin/streptomycin (Invitrogen) (0.2 ml per well) on 96 wells plates (2.5 x 10^4 cells/well) coated with Cultrex poly-L-lysine (Trevigen). At days-in-vitro (DIV) 5, cells were treated with cytosine P-D-arabinofuranoside (AraC, 2nM) to inhibit glial cell division and infected at DIV5 with 10-40 x 10^3 (100-400 ng/well) viral particles containing either a mock or PM20D1 version of the plasmid. At DIV12 media from 96 wells was collected and measured using the Aβ 40 ELISA kit, human (Novex). PM20D1 contains a N-terminal secretion signal peptide. It is actively secreted and can be detected in culture media (Figure 14a). After PM20D1 overexpression, a significant decrease of soluble Aβ has been observed, with respect to control-infected neuronal culture (Figure 14b), indicating that PM20D1 acts as a direct or indirect Aβ-degrading enzyme. Following the above described surprising observations, diagnostic methods to determine a state of AD are here described. A novel therapeutic approach is also described. These methods comprise the steps of placing a sample onto a substrate labeling the sample to identify at least one epigenetic marker and/or genetic variation; performing a multivariate statistical analysis to produce an output value; comparing the output value to a reference value; and determining a state of Alzheimer's disease, wherein one of said epigenetic markers is PM20D1 promoter DNA methylation and/or one of said genetic variation is at least one PM20D1 mQTL-associated SNPs. Preferably, said SNP is at rs708727 and/or rs947211 and/or rs708730 and/or rs708724 and/or rs823130.

In a more preferred embodiment, said epigenetic marker is PM20D1 promoter DNA methylation, wherein an increase in methylation levels is associated with an AD diagnosis.

In a further preferred embodiment, said SNP is at rs708727, wherein the AA haplotype is associated with an AD diagnosis and/or at rs947211 wherein the GG haplotype is associated with an AD diagnosis and/or at rs708730 wherein the AA haplotype is associated with an AD diagnosis and/or at rs708724 wherein the CC haplotype is associated with an AD diagnosis and/or at rs823130 wherein the TT haplotype is associated with an AD diagnosis.

In a further embodiment, it is here described a method comprising the steps of identifying PM20D1 RNA expression in a sample; determining the expression level and comparing the value to a reference value; and determining a state of Alzheimer's disease. Optionally, said sample is genotype-corrected and, wherein said sample is a G rs708727 carrier, an increased PM20D1 RNA expression is indicative of AD.

It is here claimed a method for detecting the presence or absence of a epigenetic marker in PM20D1, or the expression levels of a gene product thereof and/or of genetic variations in the nt SEQ ID No.5 on chromosome 1 indicative of AD in a subject, comprising:
(a) contacting a sample from the subject with a reagent capable of detecting the presence or absence of an epigenetic marker in PM20D1 and/or the expression levels of a gene product thereof and/or genetic variation in the nt SEQ ID No.5 and
(b) determining the presence or absence of the epigenetic marker and/or genetic variation, wherein the presence of the epigenetic marker and/or genetic variation indicates that the subject is afflicted with, or at risk of developing, AD;
Moreover, it is here claimed a method for diagnosing or prognosing AD in a subject, comprising:
(a) contacting a sample from the subject with a reagent capable of detecting the presence or absence of an epigenetic marker in PM20D1, or the expression levels of a gene product thereof and/or of genetic variations in the nt SEQ ID No.5 on chromosome 1 and
(b) determining the presence or absence of the epigenetic marker and/or genetic variation, wherein the presence of the epigenetic marker and/or genetic variation indicates that the subject is afflicted with, or at risk of developing, AD
Preferably, said at least one genetic variation is a single nucleotide polymorphism (SNP), an allele, a haplotype, an insertion, or a deletion, more preferably is a SNP at rs708727 and/or rs947211 and/or rs708730 and/or rs708724 and/or rs823130.

Preferably, the genetic variation is a AA allele at rs708727 and/or a GG allele at rs947211 and/or a AA allele at rs708730 and/or a CC allele at rs708724 and/or a TT allele at rs823130.

In a further preferred embodiment, the genetic variation is a SNP at rs708727.

Still more preferably, the genetic variation is accompanied by an epigenetic marker that is an increased PM20D1 promoter DNA methylation.

Preferably, for the aim of the present invention, the sample is selected from one of cerebrospinal fluid, blood, serum, sputum, saliva, mucosal scraping, tissue biopsy, lacrimal secretion, semen, or sweat.

The presence of the one or more genetic variation is carried out by a process known to the man skilled in the art, preferably selected from the group consisting of direct sequencing, allele-specific probe hybridization, allele-specific primer extension, allele-specific amplification, allele-specific nucleotide incorporation, 5' nuclease digestion, molecular beacon assay, oligonucleotide ligation assay, size analysis, and single-stranded conformation polymorphism. In a further embodiment, electrophoresis, chromatography, mass spectroscopy, proteolytic digestion, protein sequencing, immunoaffinity assay, or a combination thereof are employed.

Preferably, nucleic acids from the sample are amplified prior to determining the presence of the one or more genetic variation.

In a further preferred embodiment, the diagnostic method further comprises subjecting the subject to one or more additional diagnostic tests for AD selected from the group consisting of screening for one or more additional genetic/epigenetic markers, administering a mental status exam, or subjecting the subject to imaging procedures.

In a further embodiment, it is described a method of aiding prognosis of a sub phenotype of AD in a subject, the method comprising detecting in a biological sample derived from the subject the presence of a SNP at rs708727, wherein the G rs708727 carrier are characterized by increased expression levels of PM20D1 in a biological sample derived from the subject as compared to one or more control subjects.

In a further embodiment, it is here described a therapeutic target for the treatment of AD, wherein the therapeutic target is PM20D1. In an embodiment, a viral vector codifying for PM20D1 is administered to a subject in need thereof. Alternatively, a recombinant PM20D1 protein is used.

The described approach is particularly suitable on a sub phenotype of AD characterized by a SNP at rs708727, wherein said sub phenotype of AD are subjects G carriers at rs708727. In a further embodiment, the present application describes and claims a set of molecular probes for diagnosis or prognosing AD comprising at least a probe capable of detecting directly or indirectly at least a marker selected from the group comprising: a SNP at rs708727 and/or at rs947211 and/or at rs708730 and/or rs708724 and/or rs823130. Preferably, said set further comprise one or more probes capable of detecting directly or indirectly a DNA methylation on PM20D1 promoter.

### Material and Method

### GWAS data

GWAS public data were obtained from NCBI GEO database (phs000168.v1.p1 NIA Late Onset AD and National Cell Repository for AD Family Study). Selected SNPs were extracted using PLIN v1.07 software (Purcell S et al., PLINK: a tool set for whole-genome association and population-based linkage analyses. Am J Hum Genet. 2007; 81(3):559-75) and the relation with AD by fitting bayesian univariate logistic regression using bayesglm (Gelman A, Su YS. 2013. arm: Data analysis using regression and multilevel/hierarchical models. R package version 1.6-09) with phenotype as outcome and asset point and interval estimates coding genotype as categorical variable and the allele-dosage test (p for trend) coding genotype as an ordered factor. Hardy-Weinberg equilibrium and allelic test was performed as previously reported (Guedj M et al., A fast, unbiased and exact allelic test for case-control association studies. Human Heredity 2006, 61:210-221). Linkage-disequilibrium data were obtained from SNAP Broad Institute database (https://www.broadinstitute.org/mpg/snap/ldsearch.php) and UCSC tracks for HapMap Release 24 CEU recombination map and HapMap Linkage Disequilibrium -Phase II from Phased Genotypes. Layered H3K27ac profiles were obtained from UCSC H3K27ac7 cell lines ENCODE track.

### EWAS data

EWAS public data were obtained from NCBI GEO database (GSE45775 and GSE57361) and analyzed using R software (http://www.Rproject.org) as previously described (Sanchez-Mut JV et al., 2015 previously cited). Briefly, GSE45775 Infinium HumanMethylation27 BeadArray 27K (Illumina) data were quantile normalized using lumi package and analyzed with Genefilter package (Bioconductor). DMRs were defined by two or more probes consistently reporting changes of DNA methylation. GSE57361 whole-genome bisulfite sequencing data were aligned using Bismark software (Krueger F and Andrews SR, Bismark: a flexible aligner and methylation caller for Bisulfite-Seq applications. Bioinformatics. 2011; 27(11):1571-2) and analyzed with SAMtools (Li H et al., The Sequence Alignment/Map format and SAMtools. Bioinformatics. 2009; 25(16):2078-9), Bedtools (Quinlan AR and Hall IM, BEDTools: a flexible suite of utilities for comparing genomic features. Bioinformatics 2010, 26(6):841-842), Tabix (Li H Tabix: fast retrieval of sequence features from generic TAB-delimited files. Bioinformatics 2011, 27(5):718-719), and ggplot2 packages. DMRs were identified by seeking regions with more than five consecutive CpG sites consistently located outside the 95% confidence interval of the smoothed methylation profile.

### Brain samples

Post-mortem tissues were obtained from the IDIBELL Biobank. Samples were dissected and characterized for Braak stage before further examination. DNA and RNA from grey matter samples of frontal cortex were extracted for subsequent experiments. Only samples with RIN > 6.5 according to the RNA quality test developed using the Agilent 2100 bio analyzer were included in the study. From these selected samples DNA and RNA from grey matter of frontal cortex (Brodmann area 9) were obtained, from 22 controls (Braak 0---II; 32% female; age 64 ± 3 years, mean ± SEM) and 23 Alzheimer's disease (Braak V---VI; 43% female; age 77 ± 2 years, mean ± SEM), wherein samples were age and gender matched.

### DNA methylation

DNA was isolated from post-mortem human brain samples by phenol-chloroform extraction and bisulphite converted using the EZ DNA methylation kit (Zymo Research) according to Bibikova et al. (2009) with minor modifications. Primers for bisulfite cloning/sequencing were designed using the Methyl Primer Express, version 1.0 (Applied Biosystems), and PCR products cloned in the pGEMT-easy sequencing vector (Promega) and Sanger-sequenced. Primers for pyrosequencing were designed using PyroMark assay design program, version 2.0.01.15 (Qiagen), and pyrosequencing reactions analyzed using PyroMark Q24 System version 2.0.6 (Qiagen) following manufacturer instructions.

### RNA expression

Total RNA purification and DNase treatment were performed using TRIzol (Invitrogen) and the Turbo DNA Free kit (Ambion). RNA was reverse transcribed using the Thermoscript RT-PCR system (Invitrogen), and PCR run using StepOnePlus RealTime PCR System (Applied Biosystems) and SYBR Green PCR MasterMaster Mix (Applied Biosystems). PCR efficiencies were calculated using standard dilutions and the LinReg software (Ruijter et al., 2009) and three housekeeping genes (GUSB, RPL38 and TBP) for normalizing PCR signals.

### Chromosome Conformation Capture

Chromatin conformation capture protocol (3C) was adapted from Court et al., 2011. In brief, cells were crosslinked 5 min with formaldehyde 2%, blocked 5 min with Glycine 125 mM, and lysed using an hypotonic buffer solution (Sucrose 0.25 M, KCl 25 mM, MgCl2 5 mM, TrisHCl 20 mM, pH 7.5). Nuclei were resuspended in CutSmart Buffer (New England Biolabs) and permeabilized with Triton X100 0.2 %. Chromatin was digested overnight with HindIII (New England Biolabs) and ligated overnight in diluted condition with T4 DNA ligase (New England Biolabs). Crosslinks were then reversed overnight at 65° C with 200mM NaCl and protein K treated. DNA was phenol-chloroform purified, ethanol precipitated, and resuspended in TE. BAC RRP11-219P13 (CHORI) was used for assaying all potential 3C products and primer PCR efficiencies, and efficiency of proximity ligation and DNA concentration for normalizing 3C signals.

### Chromatin immunoprecipitation assay

ChIP experiments were adapted from Graff et al., 2012. Briefly, cells were crosslinked 10 min with formaldehyde 1 %, blocked 5 min with Glycine 125 mM, and cytoplasm lysed using cell lysis buffer (HEPES 5 mM, KCl 85 mM, NP40 0.5 %, pH 8). Nuclei were lysed with nuclear lysis buffer (TrisHCl 50 mM, EDTA 10 mM, SDS 1%, pH 8) and sonicated to ∼300 bp average size with EpiSonic Multi Functional Bioprocessor 1100 (EpiGentek). A total of 5-20 µg per sample and ChIP reaction was immunoprecipitated overnight at 4° C with H3K9ac (abcam), H3K14ac (abcam), H3K27ac (abcam), H4K12ac (abcam), CTCF (Millipore), MeCP2 (Sigma), and PolymeraseII (abcam) antibodies in diluted condition. Immunoprecipitated chromatin was then isolated using IgG Dynabeads (Life Sciences), and washed twice with low salt buffer (NaCl 150 mM, SDS 0.1 %, NP40 1 %, EDTA 1 mM, DeoNa 0.5 %, TrisHCl 50 mM, pH 8), high salt buffer (NaCl 500 mM, SDS 0.1 %, NP40 1 %, EDTA 1 mM, DeoNa 0.5 %, TrisHCl 50 mM, pH 8), lithium buffer (LiCl 2250 mM, SDS 0.1 %, NP40 1 %, EDTA 1 mM, DeoNa 0.5%, TrisHCl 50 mM, pH 8), and TE buffer (TrisHCl 10 mM, EDTA 0.25 mM, pH 8). Chromatin complexes were eluted with a solution of NaHCO₃ 0.1 M and SDS 1 %, and decrosslinked overnight at 65° C with 200 mM NaCl and proteinase K. DNA was phenol-chloroform extracted, ethanol precipitated, and resuspended in 50 µl TE. PCR was carried out in triplicate using SYBR Green PCR MasterMaster Mix (Applied Biosystems). Thermocycling conditions: 10 min 95°C; 50 cycles, 15 s 95°C; 1 min 60°C. Fluorescent signals were acquired by the StepOnePlus Real Time PCR System (Applied Biosystems). Values were normalized to total DNA input.

### Cloning

Human PM20D1 (AK057131.1) was cloned from a library of oligodT cDNA derived from SH SY5Y neuroblastoma cells. Rs708727 enhancer was cloned from immortalized B cells DNA. For PM20D1, forward 5'---AAAAGAATTCGCCACCATGGCTCAGCGGTGC---3' (SEQ ID No. 1) and Reverse 5'-CAAGGGATCCAGATGTCGGGAGGAAGGG---3' (SEQ ID No. 2) primers, containing EcoRI and NotI digestion sites respectively, were used. For rs708727 enhancer, were used the forward 5'-AGTAGGTACCCTTAGCTTAGCCATGTCATAGCCTTC---3' (SEQ ID No. 3) and reverse 5'-CGACGCGTCGATATTGGTTTACTCTCGTATCCTTAAAAAG---3' (SEQ ID No. 4) primers, containing KpnI and MluI digestion sites, respectively. Transgenes were amplified with high fidelity Prime STAR Max DNA Polymerase (Takara) according to manufacturer's instructions. PCR products were cloned in pGEMT easy (Promega) for amplifying the yield. Inserts were released by y EcoRI and NotI, and KpnI and MluI (New England Biolabs) digestions and ligated into pLVX-IRES ZsGreenlvector (Promega) and pGL3-promoter Luciferase Reporter Vector (Promega), respectively. All cloned plasmids were verified by Sanger sequencing.

### Luciferase assays

HEK293T cells were transfected with pGL3-promoter Luciferase reporter vector (Promega) containing mock or rs708727-enhancer using Lipofectamine 2000 (Thermo Fisher Scientific). After two days, cells were lysed and enhancer activity measured using Luciferase Assay System (Promega E1500) according to manufacturer instructions.

### Lentiviral production

Lentiviral vectors were produced in HEK293T cells using a third generation packaging system by calcium phosphate transfection with the following plasmids: pMD2.G (2.5 µg), psPAX2(7.5 µg) and pLVX-IRES ZsGreenlvector containing PM20D1/mock sequence (10 µg). After four days, medium was collected and centrifuged in a SW32Ti ultracentrifuge rotor at 19, 000 rpm for 90 min at 4°C. The pellet was resuspended in 120 µl of buffer containing 1× PBS pH 7.4 and 0.5% BSA for a 50X concentrated virus stock. Viral titer was determined using the HIV---1 p24 antigen ELISA kit (Zeptometrix Corp).

## Claims

1. A method for the diagnosis or prognosis of Alzheimer's disease (AD), comprising the steps of placing a sample isolated from a person suspected of having AD onto a substrate labeling the sample to identify at least one epigenetic marker and/or genetic variation and determining a state of AD, wherein one of said epigenetic markers is PM20D1 promoter DNA methylation and/or one of said genetic variation is in at least one of the PM20D1 mQTL-associated SNPs.

2. A method for detecting the presence or absence of an epigenetic marker in PM20D1, or the expression levels of a gene product thereof and/or of genetic variations in the nt SEQ ID No.5 on chromosome 1 indicative of AD in a subject, comprising:
(a) contacting a sample from the subject with a reagent capable of detecting the presence or absence of an epigenetic marker in PM20D1 and/or the expression levels of a gene product thereof and/or genetic variation in the nt SEQ ID No.5 and
(b) determining the presence or absence of the epigenetic marker and/or genetic variation, wherein the presence of the epigenetic marker and/or genetic variation indicates that the subject is afflicted with, or at risk of developing, AD.

3. A method for diagnosing or prognosing AD in a subject, comprising:
(a) contacting a sample from the subject with a reagent capable of detecting the presence or absence of an epigenetic marker in PM20D1, or the expression levels of a gene product thereof and/or of genetic variations in the nt SEQ ID No.5 on chromosome 1 and
(b) determining the presence or absence of the epigenetic marker and/or genetic variation, wherein the presence of the epigenetic marker and/or genetic variation indicates that the subject is afflicted with, or at risk of developing, AD.

4. The method according to any one of claims from 1 to 3, wherein said at least one epigenetic marker is an increased PM20D1 promoter DNA methylation.

5. The method according to any one of claims from 1 to 4, wherein said at least one genetic variation is a single nucleotide polymorphism (SNP), an allele, a haplotype, an insertion, or a deletion.

6. The method according to any one of claims from 1 to 5, wherein said at least one genetic variation is a SNP at rs708727 and/or rs947211 and/or rs708730 and/or rs708724 and/or rs823130.

7. The method according to any one of claims from 1 to 6, wherein said at least one genetic variation is a AA allele at rs708727 and/or a GG allele at rs947211 and/or a AA allele at rs708730 and/or a CC allele at rs708724 and/or a TT allele at rs823130.

8. The method according to any one of claims from 1 to 7, wherein said at least one genetic variation is a SNP at rs708727.

9. The method according to any one of the claims from 1 to 8, wherein the sample is selected from one of cerebrospinal fluid, blood, serum, sputum, saliva, mucosal scraping, tissue biopsy, lacrimal secretion, semen, or sweat.

10. The method according to any one of the claims from 1 to 9, wherein the presence of the one or more genetic variation is carried out by a process selected from the group consisting of direct sequencing, allele-specific probe hybridization, allele-specific primer extension, allele-specific amplification, allele-specific nucleotide incorporation, 5' nuclease digestion, molecular beacon assay, oligonucleotide ligation assay, size analysis, single-stranded conformation polymorphism, electrophoresis, chromatography, mass spectroscopy, proteolytic digestion, protein sequencing, immunoaffinity assay, or a combination thereof.

11. The method according to any one of the claims from 1 to 9, further comprising subjecting the subject to one or more additional diagnostic tests for AD selected from the group consisting of screening for one or more additional genetic/epigenetic markers, administering a mental status exam, or subjecting the subject to imaging procedures.

12. A therapeutic target for the treatment of AD, wherein the therapeutic target is PM20D1.

13. A viral vector codifying for PM20D1, and/or a recombinant PM20D1 protein, for use in the treatment of AD.

14. A viral vector and/or a recombinant PM20D1 protein for use according to claim 13, wherein said treatment is administered on a subphenotype of AD, wherein said subphenotype of AD are subjects G carriers at rs708727.

15. A kit for diagnosing or prognosing AD comprising at least a probe capable of detecting directly or indirectly at least a genetic variation selected from the group comprising:
a SNP at rs708727 and/or at rs947211 and/or at rs708730 and/or at rs708724 and/or rs823130.

16. The kit according to claim 15, further comprising one or more probes capable of detecting directly or indirectly a DNA methylation on PM20D1 promoter.


SEQUENCE LISTING

<110>  EPFL

<120>  Methods of diagnosis Alzheimer's disease

<130>  E0103124

<160>  5

<170>  PatentIn version 3.5

<210>  SEQ ID No.1
<211>  31
<212>  DNA
<213>  Homo sapiens

<400>  1
aaaagaattc gccaccatgg ctcagcggtg c                                    31

<210>  SEQ ID No.2
<211>  28
<212>  DNA
<213>  Homo sapiens

<400>  2
caagggatcc agatgtcggg aggaaggg                                        28

<210>  SEQ ID No.3
<211>  36
<212>  DNA
<213>  Homo sapiens

<400>  3
agtaggtacc cttagcttag ccatgtcata gccttc                               36

<210>  SEQ ID No.4
<211>  40
<212>  DNA
<213>  Homo sapiens

<400>  4
cgacgcgtcg atattggttt actctcgtat ccttaaaaag                           40

<210>  SEQ ID No.5
<211>  63409
<212>  DNA
<213>  Homo sapiens

<400>  5
aaaaaagtat ctctgaaaat tattttaaat attgagttga gcaaaaacgc taagttaaac      60

attttggctg tttttcctaa aatgttttaa acagctaaaa atcaatgtgt atggaatgta     120

ttcaccattg aggagatatt aaaaaaaacc tgaccatctt cacactgaat actaggcaag     180

aaagattagt accatggaaa aaaaatgcta gttttgtatt cagatctgag ctcaaacctt     240

agctctgcta tcatagctct gtaactgtaa catattcttt aagcttgttt tgtcatgtta     300

catggtgata aagaaaaata cttgttacag ggttgtaata aggataaaac aaagtgaaaa 360

cctctagcac agttcctgac tgcatttttt ctcaatgttt gttgaaaatg aaaacatttt 420

aaacatttaa aatgaaaatt ttaaacactg taattttcag gtaattaatt acatgtatgt 480

tgagcaaaag tgaatacttt tcttataaaa gtaactttcc ggccgggcgc ggtagttcac 540

gcctgtaatc ccagcacttt gggaggctga agtgggtgga tcgcctgagg tcaagagttc 600

gagaccagcc tggccaacat ggtgaaaccc tgtccctact aaaaatacaa aagttagctg 660

ggtgtggtag tgggcacctg taattccagc tactctgagg ctgaggcagg agaatcactt 720

gaacccggga ggtggaggtt gcaatgagcc gagattgcac cattgcactc ccgcctgggc 780

aacaagaggg aaatcccatc tcaaataaat aaataactcc cccattattc aggcattgta 840

aattatgtat ctttgatggc atgtctttga aggaattatg tctttttaga ggaaactgac 900

aggtaatttt aaaaagtata gatccaaggt taataaactc acttctctct ctctctctct 960

cacacacaca cacacacaca cacacacaca cacactagag aataagagaa tgagccagtc 1020

tcatctacaa ccatattaaa cttgtgtctt catgtgcaca tttggtaaca cattttgaac 1080

taccttgaat tttcaagttt ctagtttttc tctttagctg aggacttctt gaaaccaatt 1140

gttttggggg agagaatatt tttagaatcc aggcatggcc caaacaattc tctttgaaaa 1200

gaaagcaaac tggaatcctg ggaaagccgt taattctcaa cactacttat aaacatgaca 1260

aacaataaaa taaattctat aacaacttgt aagagcttat gttttcacat ctaaattttc 1320

aatggtaagg ttccttacaa tctcaagtgt taaacaaaag gtatcaagca cacttaggtt 1380

acaaggagaa gcaaaaagat tggtttgagc aacctggatc actttcaata agggaggagg 1440

atctctggag agtgtaacag ctgaatttca agaaatctgg tttctagttt gggcaagtca 1500

gtaaatctga gctttctttc tcacctctta caagtaatgg cattttcctt ccttcctcac 1560

aaagttgact catgaaagca cttaaaaaag ttaaaaatgt tacatactat gttctttaat 1620

cagcactaat aacattaata ttgatcgggc agaaggtaca gcgatcatgc gggtggggac 1680

cagaattggg aattgggaat tgggaaatct aggttctcag cccaacattt gcacctgcta 1740

gttcgtgatg caaggcacct cacttatcca ggtccccatt aaactaggtt taataaagtg 1800

agtttgccta aacctcttcc tagctccaag gctaatttta agctattaat ttgaatccaa 1860

ggcctaaaat atttgagctc tcatttaaca gtcggccact gcaaagggta aagacagcaa 1920

tcttgtatga acaaagttaa acataacgtg ctagacacag gtctttgcaa aaccatgcag 1980

tagccaatag gtccaccata ccacccttca tttggaaatt gatgctaagg tgccataggt 2040

acatttccaa atgctcttaa aatgtttagg ttcatattaa aataaaaatg gatatttatg 2100

gtacaatgag aaccagaaaa actgcaaaat tatacacagg cctaagggca gaaatacccc 2160

aaaagacgac acagtgacaa tattttccaa cttgtgcttt tttgttttta aaacaaggct 2220

gatgattctg aaatcaggtc aatgtctatc acttttttcg ttgatgttaa atgcactaag 2280

ggaatataga ttcttcaatt atactttgaa acaacatcat ttccctaagt caggactttt 2340

ttattgcaaa atttaacaaa ccaccaaact caagtttaat tatttctgct ttattattgg 2400

tcatgaataa agacttcttt taaaaaataa ttacaaagaa tttatgacta aaatatactc 2460

cgtaaccaac ttgctctgaa attaataatt tgaaaatggc aaagaaaaaa aaaaacagcc 2520

cacaacatat taacttcact cttcaattag gggaaataaa caaggtcaaa attagtttaa 2580

acaacatact gagtaataaa aaagttgtgc agaagtccca atgcctccaa aaaactgata 2640

gaatgtgctt aatatatata tccccacatg gtaacataaa aaatccaagt tttccagtaa 2700

aaatgttctg ttcatgttcc aatacctaaa tctgggaaaa caaattcagg cagatgagat 2760

aagaacgtca ccacaataaa ataaagtcat caaaacttag tgaactaaaa tttaaaaggt 2820

agaggaatta tttttcctaa tttgacctaa tttgaccaca cagtaattac ttccagaaga 2880

caaaagatcc aatccaggta aaataaatct ttattttatt attttacctg atcctaaaga 2940

aaatattcta cgtgagttga tggatacatt ggtcaagttt tatgccaaaa aaattacgca 3000

aactatactc tgaaagagct atccagactc cttgccaaaa ggccagcata actttcagag 3060

tagagatcgt gagttaccac agatgctgat tgaaacagag ctaacagcag ctttctcctc 3120

attcccaccc cgtgtgtagg agtacgcagt ctcataccac caccagttgg aggatgatct 3180

agtctggaga cagaacggtg tagaacaaaa cccttgccag ggtcccaaac ctctctttag 3240

aactctaatt aaaggtactt ccgtttccca cacaacttgg aatgaagggg aagcaacgac 3300

tgttaaaaca tttaaaaact aagcctttat ctatttattt agaaaacaca ctatgcaaag 3360

aagccaattc cttacattaa aaatatttat ttgcaacagc tctccttttt aaggcctcaa 3420

cagattggac taaaccacac ccctccataa atggcacatt ccatacaata gcctggagaa 3480

tggcatagga cgattccacc gacagctggg ggaatagcaa acttgttgaa attccgagaa 3540

tgaagtctga tggttacccg aacctagccc ttttattact aaagtcactc ggggacaaga 3600

gggttacaaa aatatcccga gacgcgtacg cgggtgagag tggaacgaga accacacggg 3660

atttgggccg tgctgttaac ccgcacctcc ctgagtacgg acatttcccc gaggaaggac 3720

atttctagtc ggagagaaag agcgaacttt ggggttaact ctctcaaaac tctgaggtgg 3780

ggagagaggc agcagcccat tattttccag gatccagtcc cgaactctca cttttgcccg 3840

gggggaaaaa aacgggtccc gcccacgctc ggcccgatcc gggaggcggg gcctgcagcg 3900

accccgaagg acagaggcgg ggccggttgg cagtgctgcc cctccccctt tgccaggaac 3960

acccccgtcc gctacagaga gggctcaggg cccccacccc cgcgccagtc gctgggggaa 4020

ggggcaatac gggtgcctcc gcccctcctc gctgaaggtg gggagtggag ctcgagtccg 4080

cccgcctccc cgcgctggct ccaaggggac ggccccgagg aggtggggcc tcttgggggc 4140

gctgacttca cacccctccc cctccggccg ctggcggctc agggacaccc cgcccgctct 4200

agtgcggtgg gtgggggagg cagctgagca gaccggagcc ctgcagcggc gcggggcggg 4260

gaggggcgcg cgcacgggcg cgcagccacc acccccgcgc gccagcaggg ccccccacgc 4320

tcaagggtgc gcgcgggccc cgaaagggag gaggccgcgc gcggcaccgg ggatggcgga 4380

ctctagcctt ctgtccccca ctcttttcac cactcgcccc catccccctc caacccgctc 4440

caggcctcag actccgcact gacctgacag gccgcgacat gttcgctgtc gaaacaggac 4500

cgagtcgaga agccaaagac caggaccccc cccaccccgc gcgctcggcg ccccaccccc 4560

cccgaacttc agccgatggg accgctgctg ccgaaccccg agctgctggc ttctcaaact 4620

ccgctgctct ttggttcagg gctcctggaa cagacgagcc ccccgctccc ccgtctcttc 4680

aaaatggatg aatcaaacca gccgaaaatg cgccaaagcc gccgtgcaac caaacccact 4740

agggtttgtg cgctcctccc caacacgcct gctcattgga gacttctgcc agatgcgccc 4800

agatcattag tccgtttgaa tcatcacgtc ttccaggccc cgccctgctc tctgataggc 4860

tccaccttca ccgtgggggt cctgttagtc aagatgctct gagagcgcac ggccgcaaaa 4920

caaaaatggt cgctgcttag cccgccccct ggtttccact ccagagttgc agattcgtct 4980

ggggtcgtat cttagaaaga ctagaaaaaa agatttatgt agggttcagc gttgctcagt 5040

gttagtttaa tggtgtttca tctcgtctgc cttgatcacc ttgaaaatcc tctaatagca 5100

aaacccgtgg gagaggcagc agattcgtca ctgcggggct gctacagaat tggagggaaa 5160

ttccttaggt tcaaaaaatt tgaccttaaa cctacaagcc ccttaccctg gcttcggcat 5220

taaccctcag ggaccaaatg aggctgctgg agtagagagg caggatcata atcaaggaat 5280

atcttcaccc ccaattacca ttctccattc tggaccttga gatccacttc agggtcatta 5340

aagtcccagt ctggtgcatt actattttaa gaaaagagtc aggtgtacaa taactaagag 5400

catgagctca ggggccacaa ggtatttcag gtggaatccc aggtccacta cttaatggtg 5460

gtataacttt ccttctctgt gcctcagttt tctcatctgt aaaatgggaa acaagggtac 5520

ctaactgcat aggcttatta gtattaaatg agttgatata tctatcaaaa gtctttagga 5580

tagtctctgg ctcacagtaa gtgctacgta agtgttaaat ccaagtttta aaagatgatt 5640

ccgtccctgg gtagagatgg gtggagctgg ctcaagaccc tatgaattct attgatttac 5700

ccttgcactt tgaaaaacaa aacaaagact cttcgaagtc atataactga aagtaacgca 5760

catcatttca cttaaatccg tgagcatttg ttggatgcct aatactctca aaggcagagc 5820

tcaggattcc tatgtgaatg gctaaggctg agtctgctat cgaaaataga cgtcaatccc 5880

tgtcacagtc aaagcgatca tttttgtttg gtggctgtgc gctctcagag catcttgact 5940

aacaggaccc cacggtgaag gtggggccta tcagagagta gggtggggcc tggaagacgt 6000

gatgattcaa acggactaat gatctgggcg catctggcag aagtctccaa tgagcagtca 6060

gttaacaacc aattgaagat acactttcca aacaacaaca acaaaaaaca caccacatct 6120

gcctcctgaa agtctccctt tgtaaaatct ggcttcccaa aaagaagaga atagcacagt 6180

ccaccagtga agcattaaac aaattaatat cttttgtctc ttccatgtca ttccaccctc 6240

tgtccctctt atttttattt ttaattttta ttttttcgag acggagtctc actcactatg 6300

ctccccaggc tggattgcag tggcacgatc tctgcacact gaaacttcca ccacccgggt 6360

tcaagcgatt ctcctgcctc agcctcccaa gtagctggga ttacaggcgt gctccaccat 6420

gcccagctaa ttttggtatt ttattgtgga gatggggttt caccatgttg gccaggctgg 6480

tcttgaactc ttgacctcaa gtgatctgcc tgccttggcc tcccaaagtg ctggcattac 6540

aggagtgagc caccgcaccc gtcccccgca atttttttaa attgtggtta aaaaaaaaat 6600

acataacata aatgtaccac tttaactgtc tctaagtgta cagttaagtg gcgttaagta 6660

cattcacatt gttatgcaac catcaccacg attcaacacc agaactttct cattttccca 6720

aactgaaact ctgtgcccat taaacactaa ctccccactt tcccccttcc ccatagccgc 6780

tggcaaccac cattttactt tctgtctgta tgaacctgac tgctctagga atctcatgtt 6840

agtgtaatca tacagtattt gtccttttgt gactggctta tttcacttag cataatggca 6900

tccaggttta tccatgttgt agcaagtgtc aaaatttcct ttctcttaaa ggccaaataa 6960

tattccactg tatgtatatg ccacattttg tttatccact catccatcaa tggacacttg 7020

ggttgcttcc accttatggc tattgtgaat gatgttgctg ggcaagtggg agtacaaata 7080

tgtttgagta cctgctttcc tttctggata tatacccaga aatggaattg ctagatcata 7140

cagctattct gtttaatttt ttgaagaatt gccgtatgga ttctccatca gctgtatcat 7200

tttacactcc taccagcaat gtgcaagtgt tccaatttcc ccatgtcctt gccaacagta 7260

agttttttac aatagccatc ctaatgagtg tccatatacg gttttttttt ttttttttga 7320

aacggagtct cgctctgttg cccaggctgg agtgcagtgg tgcgatctct gctcactgca 7380

aactccgcct cccaggttca cgccattctc ctgcctcagc ctcccaagta gctgagacta 7440

cagatgcccg ccaccacgcc tggctaattt ttgtattttt agtacagacg gagtttcatt 7500

acgttggcca ggctggtctc aaactcctga cctcaggtga tccacccgcc tcggcctccc 7560

aaagtgctgg gattacaggc atgagccacc acgcccagcc tttttttttt ttttagatag 7620

agtctagccc tgtcacctgg tctggctcac tgcagcctct acctcccggg ttcaagcaat 7680

tctcctgcct tagcctcaga gtagctggga ttacaggcat gcgccaccac acctggcttt 7740

ttgtattttt agtagagacg gggtttcact atgtttgcca ggctggtctc gaactcctga 7800

cctcaagtga tccacccacc tcagcctccc aaagtgctgg gattacaggc gtgagccacc 7860

gcgcccggct ccatatatgt tttgactaag tgtattacca tccccactat tgtcagaatg 7920

ccagggcaga aacttgggtg gcagagtgat caccaagtgg ctttggtgga ggctctttta 7980

ttaggccaaa ttgttattat tttagtaaac atcattattt ggtattgtga gatggggcag 8040

aggaattctt gttactcatt tattaggtag tgaacagaaa tgaccatagt caacatttgc 8100

aggcaggtgg aaagttcaga ataggtcttc aggttgttgg cctgatagtg tagtgcaagg 8160

ccagtataca aatggccact gagtgaagac tctcctcctt ccaagttcag cctcatatcc 8220

tacctttctc ttcactgcag cttcagctaa catcttcctt ccatttgctc atttattcac 8280

ttagtattgc aatcaacatc gtctctcagg gaagaaataa cttaagactt gaacttcgag 8340

tttacttttt agtggacaag tacagaacta tggccagtag tgccaaaaga acgtggaatg 8400

cagtctccaa ggtcagttcc ctctaatctg taccccagtg atctgccctg tgtacctgtt 8460

ttaaccacaa tcagtttgct ggaaactgaa gaaggacact gatcatagga taggtcaggt 8520

tttagaagca ctatctgata accccgaagg gacaatatgt gtaataaaac taggagaaac 8580

tatgaaaaca gaaaagcctc aggagacaga aaaggcggaa ttattccttt tccctggaaa 8640

tgaaatctca tggcctttta aacacagtgc ttaattttcc cctgtgattt ttagttatgt 8700

tttgagaagt accattttcc ttctccttca catatgttag acagtaagaa ctggcagtgc 8760

tgggatggta agactgatgg gttttccaca tacccaacaa aaacccattt ccatcaaggg 8820

ctacactata gcagctgaga gcttcataaa aatttttcag cattgtgtgc tgggcacttc 8880

cagttctgtg atctcatgaa agtcaaccaa ccaggtctaa agacagagac tgcttccaca 8940

gagaacagga ccacagaagg gtaccactct ttggagatgc tgccaggaag ttaaagaaac 9000

ctccaactct ttttttaaaa attttttatt tttaaatctt ctacctccaa cctgctggct 9060

ctctctgtac taccactcta gaagacagga tgggatagaa gaactgtgtg tattaggtct 9120

gtctctctac aaccccagag actgagtagc ctgcttggag atagatccgt gattagagga 9180

aattttccct tgtcttcttt ttctcaagtg tgtatttctt tgctccagat tcccatcttc 9240

ctctctactt tgaatattaa gagctaagct taggtgattt tgtactacta aaacaataaa 9300

gatgtacaac tcaatatcca gtttaaattg aagaaaaata caacaagact aacttactgt 9360

tttgctggac acaaaatcca tagaaaactt gtcattgtaa cactgaggga gagacagctt 9420

tatctatgca aaccactgac aaatattgtg aggtgaacac tgctgaaaag cgaggcccca 9480

aggtagaaca aaaagagcac tgaacttgta atcaagatta tctgtctctg agcttgtaat 9540

ttactaccta tgtcctttag caaatttcaa actccctgtg cctcagctcc ttcatctgtg 9600

aaatggagat aattatactg gtccttaccc acttcacaag gttagcataa taacaagaaa 9660

aaaaaaaagg gatcacttgc gaaagtagtt tgtagactgt gaagcactat gagcggtaga 9720

atgggatcac attcgcttca ctaaaggaaa accccaaatt ccgatagctg acttattcct 9780

ccaacagaat acaaccatcc ctgaatactg gtaacagtta aaaaaactgc tcagcagggc 9840

gcggtggctc atgcctgtaa tcccagcact ttgggagacc aaggtgggcg gatcacgagg 9900

tcaggagttc gagaccagcc tggctaacat ggtgaaatcc cgtctctact aaaaatacaa 9960

aaattagctg ggtgttgtgg tgcacacctg taatcccagc taccagggag gctgaggcag 10020

gagaatgact tgaacccagg aggtggagat tgcagtgagc cgagatggtg ctactgcact 10080

ccagcctgga tgtcagagca agactccatc tcaaaaaaac aaaacaaaac aaaactgctt 10140

acagcttcca gatgagatga agaaaaggtg cagtaatgag gagtgatgac catcagcttg 10200

cagcactgcg atacactttc tgtttcatat agagagacaa tgacattctc tacctcccaa 10260

ctattcctag gagaattaag ctgctccaac catgaccata taggcatgat ttcaccaact 10320

agtgccagcc tttctacttc tgtattggtt ccagctcggt atccctaact gcctaacagg 10380

tatttccact tggatgtcca caagctcaat ttatttatgt caaaaaaaat catctttccc 10440

acccacatta ttttcctgac tctattcctg tcctctgtat cattttcccc tgtcagtttt 10500

attgaggtat aattgacaaa taaaaattgt atatatttaa gttgtacatt gtgatgtttt 10560

gatacattat gaaatgatta tccaatcaag ctaattaaca tgtccatggc ctcagttatc 10620

tttttttggt gtagtgagaa tacttaagat ctactctcca agcaaatttc aagtatataa 10680

aatattaact atagtcacca tgctgtacat taggtctcca aaacctgttc atcttataac 10740

tgcaagtttg tgccctttga ccatcatctc ctcatttttc ccatcctctg acccctggta 10800

gccatcctct actctctttt tctatgagat ccacttttct gagattccac acactggata 10860

aacgagatca tgcagtattt gtctttctat gcctggctta tttcacttag tataatgtct 10920

tccaggttca tccatgtggt tgcaaatagc agaacttcaa gaaggctgaa taatattctg 10980

tcgtgtgtat gtaggtatat aaacacacat atcacatttt ctttatccat tcatccactg 11040

acaatctgat tccatatcgt ggctattgca aataatgctg tgatgaacat gggcatgcag 11100

atacctcttt gagataccaa tttcattttc tttggatata tatccagaag tggggctgct 11160

ggatcatatg gtagttctat ttttaaattt tttgaggaac ctctatactg ttctccacaa 11220

tggctgtacc aatttacatc ccactaatga tgtataaggg ttcccttttt tccacctcct 11280

caccaacact tgttatcgtt tgactttttg agaatagcca tcctaccagg cgtgaggtgg 11340

tatctcattg tgttttgttt ttttttgttt ttgttttttg agatggagtt tcactcttgt 11400

ccctcagggt ggagtgcaat ggtgtgatct cggctcactg gttcaagaga gtttcctccc 11460

tcagcctccc aaggtagctg agattacagg tgcacaccac cacatctggc taatttttgt 11520

atttttagta gagacgggat tttattacgt tggccaggct ggtctgaaac tcctgacctc 11580

aggtgatcca cccaccttgg cttcccaaag tgtgcccggc tcattgtggt tttgatttgc 11640

atttccctga tgactgtgat gttgagcacc tttttcatat acctgctggg tgtttgttgg 11700

gtgtttgtat gtctcctttg gaaaaatgtc tattcaggtc ctttgtccat tttttatttg 11760

ggttatttgg gttttttttt tttttttttt gctattgagt tgtatgagtt ccttatatat 11820

tttggatatt aatgccttac tgaaaacatg gtttgcaaat attttctccc attctgtagg 11880

ctgccttttc attttgtaga ttgttttctt tgctgtgcgt tatcaccttc ctcttagcta 11940

tctgtattca aatcttggta ctactgactc cgtcttatgc ctcactgtta gtcttcaaag 12000

tggaaacctc ttgattcctt cttcattgca tttgtcaaat tcatttttca cttgccattt 12060

acattgtcac cattcatgct taaactacta taacagtctt tcttctttcc tttttctgta 12120

atctgtagta gacacaacag gcaatactac cttcctgtgg tttacagtac tcatatcatt 12180

ctcatactca aattttcaat aagtccatac agcttccaca ataaaggcta aactgtgcta 12240

gcatttaagc taccatcaga cagatttctc aagcccaaat ctaatattac tctacttaaa 12300

atacctccac gggccaggca caatggctca cgcctgtaat cccagcactt cgggaggctg 12360

aggcaagcgg atcacttgag gtcaggagtt cgaggccagc cggaccaaca tggtgaaacc 12420

caatctctac taaaaataca aaaaaaaaaa aaaaaattag ctggtcatgg tggtatgcgc 12480

ctgtaatccc agctactgag gaggctgagg catgagaatc aaatcgcttg taccctggag 12540

gtagaggttg cagtgagctg agatggcgcc actgcactcc agcctgggag acagagtgag 12600

actccgtctc aataaaaaat aaataaaatc cctccatgaa ctggggaaaa tatttgcaat 12660

atgtgtgaca aaaggttaat gttcataata taaaaagaac tttttaaaag tcacaagaaa 12720

aagacaaaat gaaaacagac ataggacaca aataggtgat tcatacaata aaaatgacta 12780

atacatttaa aaatatgatc acttgtaaca aaaaacaaat taaaaacatt ttctagcatg 12840

cacactggag gattagaaaa ggtaaattat tttaagaaaa tttaaaaaat atttcatttc 12900

ttagattggt aaaagttgaa aacagtcttg ttaaggatat ggggaaaatg gcctctcaga 12960

ccctactggt gggagtttat ttcaaagtgc atatactctg acccatttct aggagttcct 13020

aaagaagttt cacataaatt tataaatatg taaatatgtt agtttgtagc accgtttgta 13080

atagaggaaa aaggaatacc tcaaataccc atcaatagat aaaggccagg cacagtggct 13140

cacgcctgta atcccagcac ttcaggaggc cgaggcgggt ggatcacctg agttcaggag 13200

ttcaagatca gcctggccaa cacagtgaaa cctcgtctct actaaaaata caaaaaatta 13260

gccgggcttc tggtggcagg tgcctgtaat cccagctact gcagaggctg aggcagaatt 13320

gcttgaacct gggaggcaga agttgcagtg agctgagatc atgcgactgc actccaacct 13380

gagcgacaga gcgagactcc gtctcaaaaa aaaaaaaata gcttggcaac ataaattatt 13440

aatatatcca tatagtccta tgcaacaatg aaaaggaatg agctggaact gtatgttctt 13500

agatgggaac atagccaaga tacattaaaa agaaaaaagc aaactacaga aagccaagta 13560
tgagcctgtt tattttattt atttattttt ttaaggggaa tggttcatag ctaataccgt 13620
atatgtagag aaaaacctga aaggatactc aaactcttaa tcaagttatc cttcagtgat 13680
gaaattagag gaaaattctc ttatttattt gtttgtttgg agatagagtc ttgctttgtt 13740
gcccatgctg gagtgcagtg gcacgatctt ggctcactgc aacctctgcc ttctgggttc 13800
aagcaattct cttgcctcag cctcctgagt agctgggact acaggcttgt gccaccacac 13860
ccagctgatt tttgtatttt tagtagagat ggggtttcac catgttggcc aggatggtct 13920
tgatctcttg accttgtgat ccacccgcct tggcctccca aagtgctggg attacaggca 13980
tgaaccaccg cacccggctt tttattttta ttttttgaga cagagtcttg cactgttgcc 14040
caggctggag tgcagtggca tgatcttggc tcactgcaac ctccacctcc tggattcaag 14100
caactctctt gcctcagcct ccttagtagc tgggattaca ggcatgcact accacacctg 14160
gctaattttt gtatttttag tagagatggg gttttgccat gttggccagc ctggtcttga 14220
actccggacc tcaagtgagc cacccgcctc gacctcccaa agttctggga ttacaggtgt 14280
gagtcactat gcctggttcc tttttaaaaa tgtctataat ttgaatttta aaaaacagtg 14340
tttattcttg gccctactac caactagctg tgtgaccttg ggcaagttcc ttaacctttc 14400
tactactcag ttttcccatc tttggtaggt acaataatga ctcccccaaa atgtccatgt 14460
cctaattcct ggaatctgtg aatacgtgag cagcgagtaa cgttgctaat cagctgatct 14520
tgaggtggga taattaacct cgattgtccc agtgggccta aggtaatcac aagggtcctt 14580
aaacgtggaa gaggaaccag gcatggtggc tcatgcctgt aatcttagca ctttgggagg 14640
tcgaggcagg tggattgctt gaggccagaa gttcgacacc agcctggcca aatggcaaaa 14700
ccctgtctac taaaaacaca aacattagct gggcgtggtg acgcatgcct gtaatcccag 14760
ctactcagga gactgaggca tgagaatccc ttcaacccgg gaggcagagg ttgcagtgag 14820
ctgaaatagt gccactgcac tccaacctgg gcgacagagc aaggctgttt cctgcatcgc 14880
ccctcccgca aaaaaaaaaa aaaccaataa aaacaaaaaa aatgacagtg gaagaggaac 14940
agacagacgt ggtggctcat gcctgtaatc ccagcatttt gggaggctga gacaggagga 15000
tagtttgagc ccaggagttt gagacccacc tgggcaacat agcatcctct gccttggtcc 15060
ccctaagtgc ctatctctat aaaaaattag gaaaaaaaaa ttagccatgt gtgttggtgt 15120
gggcctgtga tcctagctac taaggacact gaggtgggag gattgcttga gcttgggaag 15180
tccaggctgc agcgagctgt ggtcgtgcca ctgcattcca tcctgagcaa cagactaaga 15240
ccctgtctca aaaacaaaat aaaacaaaca aacaaacaaa caagtggaag tgggaggcag 15300
aaaaggtcag agtgatgaga tatgggaaag gtttcatcag ctatagctgg cttttgaaga 15360
tgaagaaagg gaaccatgag ccaaggaatg tgggcagcct ctagaagcca gaaaaagcaa 15420

gaaaaatgat tcttccctaa aatagctgct agagaggaat gcagctctgc caacaccagt 15480

tttacccgag agagacccat tttggacttc tgacctctag agctgtaaga taattaattt 15540

gtgttgtttt aagccactta taaaagaagt aatgtttatg tttttcaata agaaaaaaaa 15600

agattaaaaa tgccctttca atagttctct acaacagggt gaaatccagg ccagatgtgg 15660

tggctcacac ttgtaatctc agtactttgg gaggccaagg ttagaggatc acctgaagcc 15720

aggagttcga ggctgcactg agctatgacc acattactgc accctagcct gggtaacaga 15780

gcaagtccct gactcttaaa aaaaaaaaaa aaaaatggag cctaaacact tcagcatggc 15840

atacagactc tcattaattt gacccaaact ccaatctcat tttctcacct ctacctttgc 15900

tatgatctgt atgttttgtg tccttccaaa attcatttct gttttttctg aggcagggtc 15960

ttgctctgtt gcccaggctg gagtgtagtg gtgcaatcat agcgcactgc agcctcaaac 16020

tcctaggctc aagccatcct ctgtcttggc ctcccaaagt gctgggatta caggcgtgag 16080

ccaccatacc tggccctgtt tttgtatttt tttgtagaga tagggtctca ctttgttgcc 16140

caggctggtc tcaaactcct agcttcaggc aatcctccca cctcggccac ccagggtgct 16200

gggattagaa gtgtgagtca ttatgcccag cctaaaaatt catatgttaa aatctatttc 16260

ccagccaagc atggtggctc atgcatgtaa tccccacact ttgggaggcc gaggtgggtg 16320

aatcacttga ggtcaagcgt ttgagactag cctagccaac atagtgaaac tctgtctctc 16380

ctaaaaatac aaaaattagc caggcatggt ggtgcgtgcc tgtaatccca gctactcagg 16440

aggctgaggc aggagaatcg cttgaacctg ggagacggag gttgcagtga gccaagattg 16500

caccactaca ctccagcctg ggcaataaga gtgaaactcc atctcaaaaa aaaaaaaaaa 16560

aaaatctatt tcccaatgtg ttggtagtag gagatggggc ctttgggagg tgactaggtc 16620

atgaaggtgg cagtctcctg actgggatta ctgcctttat gaaagacggc acggggaact 16680

tgttttgccc cttctgccat gtgaggaagc agcaagaagg tgccacttat gaagaggagg 16740

gccctcacca gacaccaaat ttgctggcac cttgatcttg gacatccagc ctccagaatt 16800

gtgagcaata catttccgtt gtttatagat tacccaaatc taaggcattt tgttatagca 16860

gcctaaacat agagaacgct acagtcctcc tgccagctcc agctataatg aatatccact 16920

attctccaaa tttcctgtaa tcttccattt ggcaagcttt tacccagctt gagcaccctc 16980

tcctttacaa ggatttcctt gacctcatcc tcgccctcca tcactacaag aagtaaagac 17040

ttggcgctct ctgcactgag acacaattct ttttacatgt ccacctgctt tctggactgc 17100

ccttcctaga caggaacgtt cttttcccca gccctgcatc ctacatacct aatgaagtac 17160

ctggcacatg gtaggtacat cactattgct tatattaatg acctaaacaa tctgccaata 17220

atctaagctt atttcccacc gctctaaaca taaaccctgg attctactgg ctccttccca 17280

aacctgcata agaattccaa acttcttttg ccccaacctg aaattctatc tcacctcttg 17340

atttttatcc atcttaaatg ctacttcaaa aactctttct tgacctcctt cttctttgga 17400

cttcagtggc ataggccttt atatccccta caactcctac tgtaatcatg gacacataaa 17460

aggtgtgtaa taaaaaatac tctgtttagg ctaggtgcag tggctcatgt ctgtaatccc 17520

aacattttgg gatgctgaag tgagaagatt gtttgaggct aggggtttga gaccagcatg 17580

ggcaacatag caagcccctg tcactactaa aaaaaaaaaa aaaaaatttt tgtttaaata 17640

tgaatacaat tatgtgaaaa aatatttgca taaaagttga ttgaaggcaa atttatgaaa 17700

tgtaaataga ggttttgttt gggttgctgg gccctaggag gtttggattt tcctttcttc 17760

cgccccctcc cccgcctccc cacccagatg gagtttcact cttgttgccc aggctggagt 17820

gcaatggtgc aatctggctc attgcaacct ccgcctccca ggttcaagca attctcctgc 17880

ctcagcctcc tgagtagctg ggattacagg cctgtgccac catgcccggc taattttgta 17940

ttttcagtag agatggggtt tctcgatgtt ggttgggctg gtctcgaact cctgacctca 18000

ggtaatccgc ccgccttggc ctcccaaagt gctgggatta caggcgtgag ccactgtacc 18060

cggcccattt tatttaacga gtaggttttt catctttcat tataaaagta ttggaaaatt 18120

tgtataccct ttaatacatt actgtttcca ccatattgaa aacttgttaa atggaaaaac 18180

ttcaaggagg cactgtagta gagtaaaggg cccagtttct ggagcaacac cacttaaggt 18240

taaaaaaaaa aaaatcacag tttgccactt gctagctgtt tcatctttga gcaaagtcta 18300

aattttttgt tcctcagttt cctcacatat ttatctcata gggtcattgt gaaaataaaa 18360

ataatccatg tgaagtactt aggtgagtac ttggtttata ccaaacactc cgattaacat 18420

tagctattgg ccacaacagt tgaagctgga atggctgtca aaaatctctg cacttgcaaa 18480

caaaaagctg atattcaaaa ttaaatgaca gtcactaagg tgacatatct tagtaagtgg 18540

aacttgtcaa ttaaacagct tgggatatgt ctgcactgca tgctattcta actgcaccta 18600

atttggcccc aaatttaatt acatgcatgt ctctatttca cacatgaaca tgttctcaaa 18660

agatatctaa cacaaaccaa gagtttcaaa acccaaaggg ctcagttctg tttctctaaa 18720

tgtctgggct tattttctaa aataggtttt tcaggccaaa tgtttcccag aaacctaaat 18780

gacagaatgg agacagagaa tgagaagact tgcagttatg ccttggctgt attacaaatt 18840

caaccagctg cctttcagtc aaatttcaag acatttgttc cctgaaacac agaatgggaa 18900

gaattgctta ctttcttatt tcaaagatag tgtaagaggc atatacaact cacagatctg 18960

agggacgaat tctatacaaa ataaatcata aaatacctct ttcctcttcc tatctgcttt 19020

ctttattccc actaacatat agttcttaat cagatgaact ttaacttgat tgcaaattaa 19080

attagactat aaaaaaaagg aatcaaaaca gtacccatca tgatgtgatg gttaatttta 19140

tgtgtcaact tgactggacc acagggcgcc caaatacttg gccaagacat tattttgggt 19200

gtttctgtga gggtgttttg gatgagatta acatttaaat tggcagactg agtaaagcgg 19260

attgttcttc ccaatgtagg tgggcctcat ccaatcagtt gaaggcctca gtagaacaaa 19320

atactacctt cccccaagta aaagagaatt ctccttcctg acaggcttta aactggaaca 19380

ttgacttttc cagcccacta gacaacagct ctgcagattt tttttttttt tgagacagtg 19440

tctcactctg ttacccaggc tggagtgcag tggcatgatt ttggctcact gcaacctccg 19500

cctcccacgt tcaagtgatt ctcctgcctc agcctgccga gtagctggga ctacaggcgc 19560

ccaccaccat gcctggctaa tttttgtatt ttctagtaga gatggggttt catcatgttg 19620

gccaggctta tcttgaactc ccaacctcaa aggatccgct cgcctcagcc tcccaaagtg 19680

ttgggactac aggcgtgagc cactgcaccc agccaagctc tgcagatttt ggatttgcca 19740

gcctccataa ttgtgtaagc caattcctta taataaatac acatatattt atatatctat 19800

atctccctat atgtttattt ctatatatct tttacttata atatttacca atctctctta 19860

ttggttctgt ttccctggag aactctaata tagaacaata aataccatta taccaaaact 19920

gcagctaaaa cttcagagaa ttcaggtcat gctggaaaac taaaggcagg cagggttttt 19980

ctaacaacaa aggtcatata ttctgactat atcctgaagt ctctggtatt tatggcagaa 20040

taaaaaaata tgtagaaata tggtgaaacc ctgtctctac aaaaagtaca aaaatcagct 20100

gggcgtgggg gcatgagcct gtagtcccag ctactcatga ggccgaggtg ggaaggttgg 20160

aggattgatt gagtctagga ggttgaggct gtagcaagcc gagatcgcac cactacactc 20220

cagcctgcgt gacagagtga gaccctatct caaaaaaaaa aaaaaaaaaa aaaaaagaca 20280

taatgcaagg ctgggtgtgc ggtggctcat gcctcctata atcccagcac tgtgggaggt 20340

agaggcaggc agatcacctg agctcaggag ttcaagacca gcctgggcaa catggtgaaa 20400

ccctgtcttt ataaaaatac aaaaattggc caggtgtggt ggcatgcgtg tatagtccca 20460

gctactcgag gctgaggtgg gaggatggct tgcatccaga aggtcaaggc tgcagtaagc 20520

tgagattgtg ccactggact gcagtccagc ttcagtgata gagtgagacc ctgtctcaaa 20580

acaaacaaac aaaacccaac tctctctata tataatatat catatatata attatatgta 20640

gaattctttc cagaatacct cattttctag tgacttcctg cctattctaa cctgcagctt 20700

tgggggttat ttggctttaa cagatgagtc acttacacag ctgaggaaat cttcctatac 20760

acagtgaacc aagaagggtg gaggggaagg ctgggtgcag tggctcatgc ctgtaatccc 20820

agcacttcgg gaggctgagg caggtggatc acgaggtcaa agatcgagac catcctgtcc 20880

cacatggtga aaccatgtct ctactgaaaa tacaaaaatt agctgggtgt ggcggcaggc 20940

gcctgtagtc ccagctactc ggcaggctga ggcaggagaa tcggttgaac gtgggaggcg 21000

gaggttgcag tgagctgaga tcgcaccact gtactccagc ctggtgacag agcgagactc 21060

catctcaaac aaacaaacaa acaaacaaac aaacaaaaag gttgaagggg aaaagtacat 21120

tgagtttagt ttccttgggc aatctggtta ctagatttta cagaattcca tggaaatttt 21180

tacatcctct aaccatggcc ttagccactg cctacaaaag tatcaggcaa gacaggcctc 21240

acgttagatg agatagtatc ctgaggaccg aagagagacc acctacagaa aaagttaaat 21300

ctggagataa caggaaacag tgagggaccc tacctggaga aagtgaatta gggaaacacc 21360

ttggggtaat tctggcgcaa tagtagtgat aaagcagcag accattcttt actgctaaag 21420

ggccttccct gacctacaat ctcctctgct ccacttattt ggtcactcag ctaagtgacc 21480

aaatttcttc tgttcaaaga agaaagtcag ggcagacact accccacagc tgtgttctca 21540

gccctccctt agggtttctg agaacactct gggggaactg ttttcttttt tgaccaaaag 21600

ccacctgagt ggttggagag gcatcttgtc tcaaatgtag gacctcctct agggaggggc 21660

tacgatatct acgttccaaa caattttgga cgctacctct tttgaatgca ccaaagaggt 21720

agcatccgag tgataagacc taattatttt tagtagcatc gcatttccat tcatgtaaat 21780

attttatttt tcctaatcat aattgctttt ctcttatgga taggggcacg aagaggaatt 21840

aggtatattt cagttccttc tttaattagt ttcaggccta taatgttcac cagttgggag 21900

agaatatcca gaagggctgg ctggctggcc agcctctaac aatgcttgaa aaggcaggta 21960

aatcctgagt cagcaaatgg gaaaagcaag cacattcagt ttttcaaaaa ggtcacgttt 22020

ccaagtagta tttggcatgt tccatcaagt actgaattgg tactaggcag atggatctaa 22080

gcaattggta ttagtaagtt aacaggtaaa cagaataata tttaccttca ttttaaaaga 22140

aggtatttaa ccacatttct cacttgtaag tgggaggtaa acattgggca cacacataaa 22200

gatgggaaca acagacactg gagactccca aaagtgggga gagagggagt aagcgttgaa 22260

aaagtgccga ctgggtgcta tgttcactac ttgggagaca ggctaagcag aaacccaagc 22320

tttagcatca cacaatatat ccatgtaaca aacttgcaca cgtacccctt gaattgaaaa 22380

tttagaaaaa ggtatttaaa aaataaagtc tccatttccg caatcctctt gagctatcag 22440

tataaaacag cactttgaca aactcacatt aaaggccaat aaccaccatg aatacaagac 22500

ttagttctca tttatttgtg attttgcagg tgttcaaacc aatgaatgaa aatcaattat 22560

ctcttcatat caatgaaatt cactatttca ctattatctt cagaatcata gtgatcaaaa 22620

ccctacctga aaatggtaat acaaaagaga actattaata caaggtttca gaagactgtg 22680

caagttaaaa taattggcat taaaacgaca cacaacaaac caccgaaggt ttggtccctt 22740

ttctctctga tatgtggcct ctgatctctt tcttagggca gtggttttca acgtctgtgt 22800

agcagaatca cctgaaaagc taacagaata tatagaaacc caggatttgt atttgtattt 22860

aaatgtaaat acaaaaggtc tgggtctctg tatctttaca aagttctcca gcaatggttt 22920

ccaaaccagg cttatcacaa tcaccaggga agctctgaaa acaacaacac attcctaggc 22980

ttcatcccta gaaattctga ttcagtaggt ttgaggtcgg gacccaaaaa tctttttctt 23040

tttgtttttt ttttttggag atggagtttc actcttgttg cccaggctga agcgcaatgg 23100

tgtgatctca gctcactgca acctctaact cccaggttca agcgattctc ctgcctcagt 23160

ctcccaagta gctgggatta caggtgcccg ccaccacgcc cagctaattt ttcttatttt 23220

tagtagagac ggggtttcag tatgttggcc aggttgatct tgaactcctg acctcaggcg 23280

atccacctgc ctcagcctcc caaagtgctg ggattacagg tgtgagccac cacggctggc 23340

tcaggaccca aaaatcttaa aacattttta aaaaatttta acaaacttcc caggtattcc 23400

cgaccagcca gatttgggaa ccactgactt aggacactta tttaaggtct atcacagaac 23460

ttcaggcctc agagtttagt aagaatatcg ataactttgg gtaggttata aatcaccatc 23520

ctagagataa tttaacaaat taaatggtct agtagtgatt tgatactaaa aagccctgcc 23580

caatttagta cttaaattac ctaatgattt tctgtctatc tactttcctc ccccaggcta 23640

ggacctgcac ataatatgtt tcctatgact aagtgcctac gtgttcacaa agttgcccca 23700

gtggaggttg ttaacttctc tcttttcttc ttccattcag attcccaaag ctgtgaggag 23760

cagacctatt tcgaagcatc acagccagct gatgtcactt gcagtattat gcattaagac 23820

atcaacccag aaacctgcaa atgggagaca ctaggcaatc ctatcatgag gattaagctc 23880

tttgtggaag aaaatgcttt cattgcatga gataggtagt ttaaaaatga ctattctttt 23940

ttcttttttt tttgagacgg agtccttctc tgtcacccag gctgaagtgc ggtggcatga 24000

tctcggttca ctgcaacctc tgcctcccag tctcaagcga ttctcccacc tcagtctcca 24060

gagtagctgg gattacagac acgtgccatc acgcctggct aatttttgta cttttagtag 24120

agacggggtt ttgccatgct gaccaggttg gtctcgaatt cctggcctca tgtgatccgc 24180

ctgccttggc ctccccaagt gctgggatta caggcatgag ccaccatgcc cggccaaaaa 24240

acttgactat tcttgatgcc aagacaagag aaacttaatt atgacgttag aagtttcaaa 24300

aatacagtga cttctgggac acttctagta gccaaaaatc cacagatgcc cagaccccct 24360

ccttctgcat tccagaatca gatgtttaca ttccaagaca gaaagaatct gaaacaacgg 24420

cctacaagaa atttgggaaa tgacatttga tctcattttc ctctcaattg gaagtcatga 24480

tatcaatgaa tatcaaatgg aaaaataagc tcagaagtag gtgagaatta tgatctctaa 24540

aacgcaggtg ctgatttcta atccttctca taaaattccg gatcacaaat tgagggctga 24600

tgagaagatc ttacaccgaa ggcactaact ggcactaatg tgagccagca acatgtgaaa 24660

ggctaatcca ggagatgcag aaatgcacat gggttccagg tgtcttttct aagtgacaat 24720

gggcctcctt actggacagt agtcaggaga caattcaaat gtacttaata aaattgtcag 24780

gtgggcaacc aaagggaaga gacttaaaag acctcccaga actgggatgg aaaataagcc 24840

aaacactact agcagcagga ccagctggag gacttggttt gtagattgat gtagtcccct 24900

tgggtggaca aagacatgat atcttctgtg gaatttctca tcatcttttc aatgaggact 24960

ctaaaagaca aaaaataagc ctgagaagct tattttggag gaaaagcaga aatggcagat 25020

gtagtctgac ttatcatcag agagtatttg gtaaagccag aaggtttaaa tgcccctttg 25080

tccccagtta tctcaggaaa gacagtatcc ctgaccttta ggggctagca ttccaatggc 25140

tcagtctgtc attgttggct cagaaataag aaaagagggt ccacagggcc acagtggaag 25200

atggatacat ctgcatgcct atcagcatga gctacttacc tcatagcctc attaatattt 25260

ttgttctcct tgactgatgt ttctgtccaa cctgtgaaac cgttctcttt actgaaccgg 25320

tcaatctggt cccggctcac tgcccaaggg gacagatcac actagcaaag agaaaaaata 25380

aaaggcagta tcataaactt cctgcaacta agaaagaata tggctcagat actgtggatt 25440

ttaaacatat ttccaccaat ggataagaac accagcatcg gaaatctata atcaacattc 25500

ttcccaaaga aagtctcacc cttggctggg cgcagtggct cacgcctgta atcccagcac 25560

tttgggaggc tgaggcgggc agatcatgag gtcaggagag cgagaccatc ctggctaaca 25620

caatgaaacc cagtctctac taaaaataca aaaaaattag ctgggtgtgg tggtgggcac 25680

ctgtagtccc agctactcgg gaggctgagg caagagaatg gcgtgaacct gggaggcaga 25740

gctggcagtg agccgagatc gcgccactgc actccagcct gagcaacaga gcgagactcc 25800

gtctcaaaaa aaaaaaaaag aaagaaagtc tcactcaccc tcaacatcct ttgaaggagg 25860

tagagatgat tttgccaact taagaagggg aaatggatgc ccagaatcag agagcatttt 25920

acttcctttc taaccacaga tagaggctag ggaccatttt ctgagattgg ccacatacct 25980

tgttggccaa gagcaggcag ggcaccggct ctccattggg tagtgtgagc ttgctgtcta 26040

ggtcctgttt ccacctctgg ctgttgctga aggtagtggc attggtaacg tcaaacataa 26100

taacacaggc agaggcatcc cgataataca atcgtgtcat agaggtgaag cgctcctgcc 26160

ctggggagaa aggggagagt tctcaaggtg acccaagagc cggcctctcc ccattccttg 26220

tagaagaggg cagtaagcat atgtgtcaga tggggccact cactttcccc ctctgactct 26280

tggttttcta tcccataaag ggataatgaa gcccaattat ttccaaggca gattccgcac 26340

ccagattgct ccagtccaaa tccctgctct gctgcatact atttgacttc gggcaaatca 26400

agctctcagt gcttcagatt tctcatttgt aaagtgaaga taaaagcagt cgcttttttt 26460

tttttttttt gaaacagttt cctgcactcc tgttgcccag gctggagtgc agtggtgcca 26520

tctaggctca ctgcaacctc cacctcccag gttcaagcaa ttctccaaaa gcagtagctt 26580

ttatgatagg gttgatgtga ggattaatga gtcagtgaat ttaaaatact tagaagaatg 26640

cttggcacat agtaaggatt cactattttt actgtatcta ttttctgggg atcttgtttt 26700

tcaccaaggt cagccttaaa cctagaaaaa gcaccctccc ctgctgtggg agtgtccacc 26760

tatagtctgg acaatccacc accttgccag cccctccatc cacatagaga ctgctccaga 26820

tctattcagg gcaaaacatc tacaaaagct ctgcacctat tcagctcatg tgtcctcaga 26880

gcagctcaat cattccagct tcagaaaaac aatttctggc tttgctgccc cattttttcc 26940

tcagagcttt ctagttaaat caaaatttct tcccttgttc ctatctagcc ctcccacttt 27000

acctgcaata tcccacagct gaagccgcac tatctcgtag tcagaccact ggagaacctt 27060

cagagcaaaa tccactgaaa atatatgtac attatacttt aataaaattt cactgtaaaa 27120

aattaatagg gataaactca aatcaaatag gcagtgaaac cctatggaca cacaactcca 27180

atcccatatg tttaacttta agccctcaat tcactgagat gagtccacat gcgtgtattt 27240

agtgtgagat ggatgattca tactcactct ccaaaggggt ttccagcttt tactctactg 27300

tcaagctcat ttatttgcta ctgtttgtct ccaagactgg gggtgggagt aactatgaaa 27360

acaattctca ttctcaggtt aaatagtttc tgggagattt agggggtttt ctcagggaag 27420

gggtgatggg ggagaagggg gaaatgagac aagtgtggtt gctttcgtat cttccacatt 27480

caatttcact gttggtttct aatcaaattg tattatgaaa agaacacacc ctggggaact 27540

ctacatccac cataaggttg caggtattat cttgggcaag aatggaataa atggaaaagg 27600

tcccagagga gtcccaagaa gaagacactg tgtggatgtt gctgaagagt cctgctggaa 27660

tctaccacca agtctgcagg tgctgagact ccttaaggct gagtaagtat tccatgggga 27720

aagccttttg gctaaggaga gaaacagagt aatttctgag gtttggaaat attatctttc 27780

acatctgtga caggaatcaa agctaaggga aatttagact aaacactgaa aaaaaaatta 27840

gccaattata ttctttgcta aaggaaaact acagcaattg ttaaaaatta actattaata 27900

gcaggccact tttaacccaa atccgtgaaa tagggaccct tatattattt aggagtggga 27960

gggcagagcg gtcaggatag agtgtctgaa actttgaacc tttaattttg ccagacttaa 28020

attttttgtt cctttgtttt tgagacaggg tctcactctg ttgcccaggc tacagtgcag 28080

tgagtggtgc gaacggggct cactgaagcc tcgacctccc cagttcaagc aatcctctcg 28140

tctcagtttc ccgagtagct gggaccacgg ctactttctt ctttaatttt tgtagaggtg 28200

ggggtcttgc catgtttccc atggctggtc tcgaactcct gggctcaagc aatcctcccg 28260

cctcagcctc ctaaagtgct gggattgcaa gtatgaacca cggtactggg ccctactggg 28320

ggttcttaag gagaatgttt taattgcaat gtaaatgttt gttaatttct ttttaaactt 28380

ccatatgaac aggagcacac attttcaatt tttccattgg tacgagtttt ccccccttga 28440

aacaagttct tttaacagag agaaataaaa ttttaagtta ctctccccca tcaccagctc 28500

ttcctagcta aataccttct aattaacaat aaggcatttc tgcctctgaa aaaatagttg 28560

agtccaaagg ccacacccgg gaaagaaatg aatgaactcg gtgtcccctt cctccctacc 28620

ttctccagtt tgaggaaaaa agtggtggga agataggtca gagaagtggt atatttcaca 28680
ttcttggaag aattccttgg ttacctttat ttaagcaatc aacggacaaa aacacagctc 28740
atcagccctc atgggcatct gactgcgtag ccaaggggct cgttcgggga ttttgtgtct 28800
tccatcccac ccacagcccc acaacaaagc tacagtcctt cagaccagtt ccgcttcctg 28860
cgttctccca ctgaagccag gttaaacacc ttgcggttcc tccctcaaca gaggggcagg 28920
actacagccc cagccggcac ctaaccagga ggccccacag aggatctcag ctgcttctgc 28980
tgattggaga gatttgccgg acagctgaaa ggctggagac ttaagttcac agtacgcctg 29040
caataaacag caattgctca tcctcatcct cccttcccta tttctcatct tcacgccttt 29100
gctcttacct ttctcctggc agtgggctca gtttgggaaa atctaacggg aaactcaaga 29160
gaggcgtgta aaccaaacac ccggcaatta caccatctag gttcccagac agtacttcct 29220
gacccgaaga aggacccaac aaccgacagc aaatccccac cccacttggg tccccagctc 29280
gagtccgcgg tcggggcctc ctcctccccc tccccctccc cacccccgag acgtctcacc 29340
tcccaccgtg gacttgtagt gtttgctgaa gctgtcctgg gaatatcgct gcaccagcga 29400
cgtcttgccc actgcggcgt cccccaccac cagcactttg aacaggtggt cgcggctgcc 29460
catggctagc ggggtgtgcg ttttagggag gcgggaagtg tggtcgggga tcgggggtcg 29520
ctcgttttaa cccctttgga tccggaccct cttcaaactg aagtgaggca ttcccaccca 29580
ccgcctgtct gggctgctct gacgagaaag caaaaaagga aactttgcac tcaacctata 29640
cgcgcttcct ccccgggcgg ccgcaacctg ggtggggcgc agggcgcgag ctccgagccc 29700
ccggctgttc gcaccttccc caccccctcc tggcctgact gccgagaaac tggaccaggc 29760
gccgcggaac ctcacccacc gggacttccc ccgagcggct ccaagtcagt gactgaatcc 29820
cagtcagctc cttacaccac tggggccaga tgatggggac cctccccttg cagcacaaac 29880
caaattgtga ggaaggagca gccggcacct cgattcccta gacgccctcc cgcgcgcctc 29940
tctctcccct tcctccgcaa gcgtcacgtg cagggttgcc tgtggcacta catttcccag 30000
ctttcctcgc ggtaaagaga accagccgag tgcggaggct caccgcgaag cctcctggga 30060
aatgtagtat attcgttatt ttgttttaag caaacctaca ccaagtttca aggcttcaaa 30120
ctccctcgac agtttggaat cttattttga aacaattcta ggtcagtaaa gatcccttaa 30180
ggggagattt gtgcaacgtg atctcactgg cctgaatcca tccccagata gttttctcct 30240
gtctttaaag gtttaactga gttaagattg aaacttattt cattagtaaa agcccaagcc 30300
tcatccatca tgctccaagt gaagtccatt tcctattcct tcaagtagat agacaagccc 30360
aggactttgt gaaatgactc atattttctg tgtgaacttg cccaatacca ggcaagttca 30420
ggaaaacttg acccaagaga ccagtctttg gaaaaaaacc tatgccaaaa attacagcat 30480
ttaatatcca gcaataacaa taaagatgga cagaaaacaa gagacagttc tccattcata 30540

cttctacaat gcctattcta gccatactac tcatgtatca cttgagctag aagctaattg 30600

aaatacactg ggactccttc ttgatttgtt gtggctgttg ttaatagata atcataaaac 30660

tagtgtttgg caaataattt taatttctca gcctgtaact ttactgacct acctgtcact 30720

tgggtagaag ttggcaggac atgagctcaa tggcaagcaa agagagctcc attttctata 30780

cttttctagg aatagaggaa gagaaccagc ggggccatgg gaaaacagcc caggaccaga 30840

cactaggaaa tctaggtctc ttgatagaga ggctatcact tatgttttgt ggcgaaagaa 30900

catcagcatg gttcacaata tattttgccc agaatgttcg ttacccaggt acaggaggtt 30960

agttcaagct actcatcttt aaggatccag ttttctctgt tcttattcgt tttgacatag 31020

ttgaccactc cttgacattt gtttccctta tgccttctat gacgctgttc tttctttgtc 31080

atccccgtac ttctctgatg attatctttt tctttcaggt ttctctccat cccatttccc 31140

gctaagtatt tcctaaggcc tgattcttgg tctctgagtc aatcattcga taaactcttt 31200

agtgaattgt ccttggtgtc aaagatctat cttcacttta tttgttcttt atctctgatc 31260

agtgatattc tgtcaagacc agctctgttg gggagaccct aacccagtgg cgctagagga 31320

attaaagaca cacacacaga aatatagagg tgtgaagtgg gaaatcaggg gtctgatttc 31380

ctttagagct tttagagctg agagccccga acagagattt acccacgtat ttattaacag 31440

caaaccagtc attagcattg tttctataga tattaaatta actaaaagta tcccttatgg 31500

gaaacgaagg gatgggccga attaaaggaa taggttgggc tagttaactg cagcaggagc 31560

atgtccttaa ggcacagatc actcatgcta ttgtttgtgg cttaagaatg cctttaagcg 31620

gttttccacc ctgggcgggc caggtgttcc ttgccctcgt tcctgtaaac ttacaacctt 31680

ccagcgtgga cgttagggcc attatgaaca tgttacagtg ctgcagagat tttgtttatg 31740

gccagttttg gggccagttt atggccagat ttgggggggc ctgctctcaa catgtccctc 31800

ttctctgatt tgcaaattga taaatgcaaa ggcagctttg tcacggtgag ctacttctcg 31860

caggagtcag gatacacatc tgcagactat acaaagacaa acaacacaga ttaaaagcac 31920

aatcatcatt gaaatcacag agcttccaag tgtttttatc cattttcggc tcctttaagc 31980

actccagttc ctggcattaa ggtcaagtgt gcctcggatg ctttaaatat ttcttctttt 32040

aattttaaat ccttatgtta agctcctaga gcgggccata tcatttgagg ttgaggtgtc 32100

actatactgc catggttcca gataatagga actcttgctg tacttcttat tatatctacc 32160

atctgaccat tttgttcaga ccagctgaac atagtgtggc cgtggcacgc agactgagag 32220

gtgcaattca agctaaacat ccccttaggg gaccaattaa taatgattcc ataggaatca 32280

ttgtgcagca tctctgcctg ttctgcaatg caatcttcct aaacaagtac gttcattttt 32340

tctaactggg tccaatcctg tttacaaata agtttttgag ggcggtatgc ctcaattata 32400

ggagcagatt tattatggta aatactgaga tcagaaagca tgtgtaactg cgtcataaag 32460

tgattgcatc caggcattat tgccagccaa gattgataaa tatacccaat aagtataatt 32520

gttctctgtg tcagccctta atgaaggaat actcacggca ctggtgataa ccgctatcat 32580

agctaccatt aacactttat ttgttcttta tctctgatca gtgatattca gctgcccaaa 32640

aggtgccccc acttggatgt cctgccacat ccttcctctt actacattgc cataaaaata 32700

gctccttttc tcagcttccc tatttctgtc actggtctca tcatttcttg cctccatgta 32760

gacttcagat tctgagcatt attatttcct ttacacatgt ccctttatac ctatttattt 32820

gtttgtttgt ttattgagat ggagtctcac tctgttgtcc aggctggagt gcattggcat 32880

gatcttggct cagtgcatcc tctgcttcct gggctcaagt aactcttgtg cctcagcctc 32940

ctgcgtagct gggactacag gcgcatacca ccaagcctgg ctaatttttg tatttttttt 33000

gtagagacag ggttttacca tgttggccag gctggtcttg aactcctgac ctcaagtgat 33060

ctacccgcct cagcctccca aagtgctggg attacaggcg tgagccgccg cacctggtct 33120

ataccctttt aaaaaaaatt cagttaccat gtctgattga ttgtttcttc agtaaatggt 33180

ttctcccact catcctctct gcttcatatt cagtaatact ttcctttttt ttttttctcc 33240

aacttttatt ttaggttcaa ggggtacatg tgcaggtttg ttacacgagt aaactgcatg 33300

ttgcgcgagt ttggcttaca gataattttg tcacccaggt aatcagcaag taacactttc 33360

ctagttcagg ctttcaccat ctcacacctg gattcctgca gtaacctgtt tattggtctc 33420

cctgaattta ttttcttttt aactcaccgt ctacaactgg acaggatggt tttcctaaaa 33480

caccattgtt tgttcttcct tgtctaccaa ctgtctttca aaaaaaaagg ccaacctccc 33540

tgaagctggt gtccaaagct ttctatgcac ctgccccggc ctacctaacc aattttatct 33600

cacccgactt caaatgggag tttccttctt ccaacaaggt gaggttcctt atgctctttt 33660

aaagcccaat gtcatttttg attttctgtt tttgtccagg atgcatcacc tttcagaata 33720

ctcttcaggt ttctttctac acatctaaat cttacttaaa tattattaag aaaatcaaaa 33780

ggaagagaaa ctatattatt cactaagtgg gagtggattg tcttaaaggt caccgaaggt 33840

tctaaaattt tagtgtgcat aagcatcacc tacctggggt acttgttaaa tacccacatt 33900

ttgattctgt agggctaggg ggtgacccag gttaatctgc atttttgaca agtactcatg 33960

tgattgctac accacattcc actttgagaa atagtacttt tgagaaatgg tgcttcaagg 34020

cctgacttaa atatctactt atttataaac atttccctta ttattgcagc ctttattggt 34080

cacctgtagc atttattttt ttattttaat tttttaattt tttttttttt tttgagacag 34140

agtctcgctc tgtcacccag gctggagtgc agtggtgcga tctgggctca ctgcaagctc 34200

cgcctcccgg gttcacgcca ttctcctgcc tcagcctccc gagtagctgg gactacaggc 34260

gcccgccacc acgcccggct aatttttttt ttttgtattt tttagtagag acagggtttc 34320

actgtgttag ccaggatggt cttgatctcc tgaccttgtg atccacccgc ctcggcctcc 34380

caaagtgctg ggattacagg cgtgagccac cacgcccggc ctatcctttt ttttgttttt 34440

gagagggagt cgctctctgg tcacccaggc tggagtgcaa tggtgctatc ttggttcact 34500

gcaacctccg cctcccgggt tcaagtgatt ctcctgcctc aacctcccaa gtagctgaga 34560

ttacaggcac gtgccactat gccttgctaa gttctgtatt tttagtagag acaaggtttc 34620

accgtattgg ccaggctggt ctcgaactcc tgatcttgtc atctgcctgc cttggcctcc 34680

caaagtgctg ggattataag tgtgagccac tgtacctggc ctgtagcatt tatattctat 34740

ttgcactttg gtcacttaac tatgtgacca atttgtaact atttaactta tataagcaac 34800

cagcgtgtct cgaagaaaac aggaagaagt aaatttttcc ttcaaaattt tgactttgct 34860

cctgatcatc tgtgaaagga actactctta gcttccaaaa tcggaaaggt cgggagttaa 34920

gatttaagag ggaagattag caaatttctg gtactccctg aaagacacag ttctcaaagt 34980

agtaatcatt tctttgatgg ggatgagaag tgtttttctt tctctcttct cttttttttt 35040

cttttttctt ttcgtttctt ttctcttttc ttttccttct tttctttctt tcttctcttt 35100

ctttctctct ctctctctct ttctttttct ttctttcttt ctctctttct ctctttcttt 35160

ttttgagaca cagtctcgct ttgctgccca ggctggagtg cagtggcaca atctcggctc 35220

actgcaacct ccatctcctg ggtttaagcg attctcctgc ctcagcctcc cgagtagctt 35280

ggattacagg cagctgccac cacgcccagc taattttttt gtacttttag tagagatggg 35340

gtttcactat gtgggccagg ctggtcttga actcctgacc tcaagtgatc cccctgcctc 35400

agcctcccaa agtgctggga ttacaggaat gagccactgc gcctggctga aaggtgtatt 35460

tcttcaacac taaattgcat ttctgatctc agtttttaag agagacggat ataaataaga 35520

gaaagagaac acacattcaa ggtgccaaac aattgatttg cacatatagt ggcccttgaa 35580

ccactcaggg gttaggaatg cctacctacc ctccagtcga aaatttgtgt gtcacttttg 35640

actccccgag aattaactac taatagccta ctgttgattg ggagtcttac caataacata 35700

aacagccaat taacacatat tttgtgtgtt atatgtatca tatactatat tcttacaata 35760

aagtaagtta gagaaaataa aatgtattaa gaaaatcatg aggaagagaa aatatattta 35820

ctattcatta agtggaagtg gttcatcata aaggtcttca ttcttggtgt tttcatgttg 35880

aatagaccaa ggaggagggg aagaaagggg agggtttggt cttgctgtct caggggtggc 35940

agaagaggaa gaaaagccct gtgtaagcgc atctgtgcag ttccaacccg tgttgttcaa 36000

gggtcaactg gtatatgttt ggaaatatag tttggaagct ggggaaaggg gtcagccttg 36060

ctctgtaact accacacttc ctgtatgtgt ctgcctttct cttttctact ttttactttc 36120

cactttttat tttgaaaaat ttcaaaccca tagaaaagtt aacagtacaa tgcatactaa 36180

tatgtccttc atcgggattt accaattact aacattttgc catatctgct ttgtctcttt 36240

gtgtgtgtgt tgaactgttt gaaagtaagt agcagacact tcaagcttaa atacttcaac 36300

atgcagctcc taataataaa attatcctac aaagccaaaa taccattatc tttttttttt 36360

ttttttttta agacagagtc tcattctgac atccaggctg gagtgcagtg gcaccatctt 36420

ggctcactgc aacctctgcc ttctgagttt aagtaattct tctgcctcag tctcccaagc 36480

agctgggact acaggtgcac gctaccacac cgagctattt ttaatagaga tggggtttgc 36540

catgttggcc aggctggtct cgaattcctg gcctcaagtg atttgcctgc ctcggcctcc 36600

caaagtgctg ggattacagg agtgagccac ctcgcccagc ccaaaatacc attgtcacat 36660

ctatgaaaat taaccttaat ccaataatat catctaacaa acagtttaca tttacatctc 36720

tctatagctc cccaatattt ttttctcatt ttctcctcac ctgtcatcca gaatacaatg 36780

aagtttcatg catggaattt ggttattcta tttctttagt ctctttcaat cttaggagca 36840

tgctgtgtcc aatacaggag ccagtagcca tgtgtggtta tttacattta aattatataa 36900

aatcagctgg gcttggtggc tcctgcctgc aatctcagct actcagggag gctgaggcag 36960

gaggatggct tgaggtcagg agttcgagac cagcctgggc aacatagcga gatcccatct 37020

ctaaacaaat tttttaaagt aattaattaa aataaataaa tgtaaagacc agctcatctg 37080

ttgcctagcc acacttcaag tgcttagcag ccatatttgg ctagcagtta ctatattgaa 37140

cagcacagac ataaactagt ttcatcatca cagaaaggtc tgttggacag tactgtctat 37200

aacaatccct tgcccttttt tttttgactt tctttttaga gtccatgcca gttgtcttac 37260

aggaagtcgc atatcctgga ttgtctgttt attgcctcaa gatttgattt aagaaacatt 37320

ttgggcaaga gccccacaca gaggatgtgg tgtgcatctt agtatgttgc tgatgtcagg 37380

tagctattag tgatgcctac actatgaatt caatgaacac ttgttgaaga aatgatctgg 37440

tgccaggcaa tctcctaagg acttgggagc ttgacttcaa ataggagaga gacagcttat 37500

ataatctcca gagcctccaa cattgtccta ggagcagagt ggaaaaatgc tttgtaacat 37560

gaagggaaga ctgaagtgac atagcaaatg gcccagcagc agcacagagg agagggacag 37620

acccagagaa gggtgaggtc aagaggctac agctgttcct cagccccaac ccccagactc 37680

acttcgatga tcataacttt ttggctgttg ctttggggag gggcaggcag cttggtgaaa 37740

tgcctgtgac ccagctaagc attcagctgg aagacttcgc agcaaggtgc ccaaggaaac 37800

cctcaggaca gagaggctgg caagttcaaa gggcagcctg ccagtcattc ctctgctggc 37860

ccgtgccaag ggtctaggcc agatttgggc ctgggagtat ctagggaaaa catcaaacta 37920

tagttttcac tgccttggtt gtcacatttg cctccttctg tggctaccct ccttccaccc 37980

caaataaagg ccagggaaag aagacaggaa aaaagtgaaa actaaagaga aaattttgct 38040

tcaaagaact ggttgtgtgg ttcccaactg tccatatggc acaggaaagt ctcatctgtg 38100

aaacaaaata aagttccctt ccaacacaga catgactgtt ctaatttcct atgttatttc 38160

aactctctag gaggtgagaa aagcagaaat tattgcaccc ataggccatc cttagttatc 38220

cactgataac ctgcccctcc ttgccaagaa cttggaagta gcattgtcct cctacccctc 38280

tcaaagataa actgtgaaat ccacatgtaa tgagaggtgc ccaggcagga ggtgacattg 38340

acccaaggta taaacacacc taaagaagga atgcactgat ccaaagaaag gtctcagagg 38400

caaagaaagg agactctgaa agaattctgg cttcacttct ttgggctctg tctggcagag 38460

ggaccagagt cttacaagaa tatctggccc tctgtcaggt atccctattg tgcagactca 38520

tgcagccaaa tataacaagg cctggcgcct gctggacttt tgcgttttaa aactaacttc 38580

tctattagga ttcctgactt tccaggtact ttctgatcat ctgccttccc ttgtggcttg 38640

cttccaggag tgaatgtgct ggtcaggctg acgtgtccgt ccagtccact gaccagctgg 38700

catctggtct tatgcacagg cctctggggg tgtgtgggtc tggcagcctc cttgagtggg 38760

atgacatctg aatgggacta ggtttctgag cctctgagga tgcagctgga ctcagggtca 38820

tagttggctt agcttagcca tgtcatagcc ttcgcccagt ggaagcctag ctgtgaaaca 38880

ggctggtcct cctcactgct cagcgttgtc cagcttcagc tgcacccatg tcacagatgg 38940

ataaaaccca gcatcggctg ggcttggtgg ctcacgcctg taatcccaac actttgagag 39000

gccgaggcgg gcggatcacc tgaggtcggg agttcgagac cagcctgacc aacgtggaga 39060

aaccccgtct ctactaaaaa tacaaaaaaa ttagccaggt gtggtggcga atgcctgtaa 39120

tcccagctac tcaggaggct gaggcaggag aatcgcttga acttgggagg tggaggttgc 39180

agtaagtcga aatcgcacta ttgcactcca gcctgggcga cagagcaaga ctccatctca 39240

aaaaaaaaca aaaaacaaaa accaaaaacg aaacaaaaaa aacctcacaa aaaaacccag 39300

catcgctcca gggctatcca cttcttcatt ccccagttcc ttcatattat taccaccact 39360

aattcaaccc acattttatc atcatcatca taaaagcttc tatttgtcga atcttctgtg 39420

tgcctaacaa ccccacagag agggcattat tgttccattt tggggaccaa aagaggctaa 39480

ttatggccac gagctgagag agctgggatc caaatctatt actatgtctg tctacctcta 39540

attacaacta tttttaaaaa ttaagctggg tgtggggatg agggaggttt cccagaagag 39600

tacagcatcc ttagcatgtt ttctgagttt cctcattcca aaggtggaaa acaggacccc 39660

tacttactga agtaaaaaaa aaaaaaaaaa aaagaatctg ccctgattgg cgaaggcagc 39720

ctggcagttg tgtctggttg gagaagcagg agaaattcta ggtaagccct gggtcatctc 39780

agaggattct ggcagccagc tgtgaccctg atacagaaaa aagataacct agatgttaag 39840

ctaagcgaga gatatagaat gtagaagcat gagcagaagc atgagtgggc acatgatgga 39900

ctattctggc tccacaggat gggagttatg agggcgagag atcagtggcc ttgttcttgg 39960

cttggacgtg ggtcccctaa gagcacttcc ggccatggtg acatctttcc ccgctacctt 40020

cttgagcctg tgtcgaaggg tggtttggtg tgtggcctgc caatcaggag actgtagtta 40080

atattccgtc tttgacacct ggggcaggtc ttaaagttaa cctcttaaag ctttagtctc 40140

cctttccatg gacttcgaat tcctaaaaac cacatacttt atttctgagt aatttgtgaa 40200

gtagttaaca atttaggtaa tatgattgaa agttatgcgc atgacagcct gtggccgagt 40260

aaaagacaga taaacatgtg ggtgtcccaa gaaggccgca atttggggct cttctaatgc 40320

aatgttctct atacaattgt tcaacatttt tttcacttaa catttgaata taatacaaaa 40380

tatttatgtt caaaacaagg actgtatatc acataaatat gactgaatag gacataactt 40440

tttttttttt tttttgagat ggagtctctc tctgtcaccc aggctggagt gcagtggcac 40500

gatctcggtt tgctgcaagc tctgcctccc gggttcacgc cattctcctg cctcagcctc 40560

ccgagtagct gggactacag gaacccacca ccacacccgg ctaatttttt tattttttat 40620

ttttagtgga gacggggttt cagtgtgtta accaggatgg tcttgatctc ctgacctcgt 40680

gatccgcccg cctcggcctc ccaaagtgct gggaatacag gcgtgagcca ccgcgcctgg 40740

ccaggacaga acatttaaac ttatttggat gtgtttttca tttaacttaa aaaaatcacc 40800

aatgactaga taatattagt cattcatact taactaaaca taaatatgac tgtaaagtct 40860

ttgaaattat ttttaaacat tcttttttgg ttggccaagc tgtcttcact tttctgcaat 40920

ttctttttct ttcttgagtt attaagattt ttgtgttttt gaaaagagag tctcaccaaa 40980

ttgcccagac tagacttgaa ctcctgagct caactgatcc tccggcctca gcctcctggg 41040

tagctgggac tataagctca taccaccatg cctggctgta gagttttttt tttgttttgt 41100

ttctaatcat tcaagccaga cattttcatg ggtgccatat taaaatattt catatttaga 41160

taatgtaata aaaatacagt acagtaaaca gcattatact atgcattatt cggtttctga 41220

agtttagcta aaggagacaa ctaagttcag gagactgtca gctgactgac ctctgaattg 41280

gtagtgctgc ctgtaattat gttataaaaa taccatcaga gataggctgg cgccttggag 41340

ccatgacttt aaatgtccat tggaagatac tatttcagta aacaattaac gttttgcagt 41400

catgtatagt gtctcataag caaaactgag ataacaggca cccatttttc tgatcgtttt 41460

agcaatgttt tatattcctt ataaaggggc accatggcca gccatttcat ctggctccag 41520

gcatagaagg cacgcactgg gctaagaaac ctgggtgtga atatatgacc tggggcaaga 41580

catttaactt ttctctgagc ctcagttttt ctcttgcaaa atggcaataa tatctatctc 41640

atagcgttat tagagaaaac attgatgtta caaatgagtc tatcacttaa aagtatatgt 41700

atagtatact atatagtgaa acactatata aataataatt aacaagcaat tatcctcttt 41760

ccattaacct ttactagcct tataaacatt gctttgaaga gttagtactg gagcacaggc 41820

tgtgataaaa ggcagatttt tgagactggc aaatgttctg cacccagtat ctgctcaata 41880

cttttgtgac atatcccatg ggggcatgtc ctgggtgggc agtggttccc gacacaggct 41940

ttgtagtcag agatacctca gtggggctgt gctctgactc tgcctttctg tatctgccat 42000

ctcagaaaaa ctgctttgtc acaaatacgt gcgtctcata aggttgtgct atcaacgtct 42060

gcgctcccaa ctccagtcat acagagctat ttgcagttcc caaaagcatc aggcttactt 42120

ccttgctcag ctcctggttc cctctgcagg acggctcttt ctcctatttt ctgctaggct 42180

aactcctatt cttctcagct tcctccagga tgtccctgac tcccaagcct tgatcaggtg 42240

cccttttcat tggcccgctc ccttatccac cctgaattta tcccatggcc agcgtactga 42300

catgacctag tcactggcct tcgtccctcc cacactgtag tattgattag gatttttggg 42360

tttagtatat accaggcacg gagcttagtg ctttgtttat tatctcactg aggtctcata 42420

atgatcctat gaaataagta ttagtactga tgaggaaact gaagctcaga aagggtgaat 42480

gatttgccca gggttataca aatacaggca gaagagctgg gatttgaacc aaggcccatc 42540

tgactccaaa ccctttgcag tcagtctatt ataagtttct aagggcttaa aggtgaacgg 42600

tgaagacaca caggaaatac tcaacaaata agagccgtca ttattttgac ccagattgta 42660

agcctcggta gggagggaat cttaacccat aaatgcttgg acccatagca caatccccca 42720

acagaggctg agttaacaca tggagaccag agtacaacct cctcgagagc aaggaccctg 42780

catctagctt cttgagtgtg tccccgtgca ccagtttctc aataaatgcc tgttgttttt 42840

tctttttttt ttttttttga ggcagagttt tgctcttgtc acccagactg gagtgcaatg 42900

gcatgatgtc agctcactgc aacctccacc tcctgggttc aagtgatcct cttgcctcag 42960

cctcccaagt agctgggatt acaggcgccc gccaccacgc ccagctaatt tttgtagaga 43020

cggggtttca ccatgttggc caagatggtc tcgaactcct gacctcaggt gatccacccc 43080

tctcagcctc ccaaagtgct gggattacag gcatgagccg ctgtgtctgg cgatgctgta 43140

ttttctatca cacaatctct gcattttaga gatgtttcct tttttttttt gagatggagt 43200

ctcgttctgt tgccaggctg gagtgcagtg gtgcaatctt ggttcactgc aacctctgcc 43260

tcctgggctc aagtggttct cctgcctcag cctcctgagt agctgggact acaggcaccc 43320

gccaccacac ctgactaatt tttgtatttt tagtagagac ggggtttcat catgttgacc 43380

aggattgtct ccatctcttg agcttgtgat ccgcccgcct cggcctccca aagtgctggg 43440

attacaggcg tgagtcaccg cagccggccg agatgtttca tttcttgtca cactttggcc 43500

cctttctact cctaattgag gagagggtaa ggcctacaga ggggcccaag acaacagttg 43560

aagctgcttt ttgatgagac atacaggaaa caccaaccat ctaaataaac gtcctcttta 43620

ttttaccaaa tataatttat cagttaagtt gacatttgtc aattcagtta tagtaactat 43680

aaataattct cttaagacac agttctgtaa ctttttaagg atacgagagt aaaccaatat 43740

tgctccttgc agccaggcca aaagtacatg cagttggtac aatcggggaa aagaaatgaa 43800

acttccatga aagtcacctt catttctttt ccctctcacc ctacagtcct cctctgctca 43860

ctcagggcct ggggcatgcc tatctgtctg ctttttaggc aaaggaggtc actccagagt 43920

cttctgggac tgcctggcca aattccagag atgaggaagg ggcgagtgac tggggtaggg 43980

gtggctggag accacaggga cacagcctta ggggcagagg agttggtttt gatgcctgta 44040

caagaagaat ctgtgtgtaa gtattttttg aggtggagga aaagatgaaa aaagtccaga 44100

gggaggagga aaaacaagcc caggctggtg ttaatgaatt ctgttggaca caactccaac 44160

tagaaaacct ctaaaattcc ttccaattct acggttctat gattccagaa ggaggcctgg 44220

aggtctgggt agaaatggag ggagagaaaa tatggtctct ggaggtctag ggcaggttcc 44280

tgggataagg tgcagtgttg gtagaaacag gatcattgcc taagccttcc cttggaggtg 44340

ggcccttgag gctacagatg gaagggtttc tgtagaagct tctgagagag aagttagaaa 44400

aagcctacat ccaaatcccc agatctgtac acgtgtgact gcggatggag gctccagttt 44460

tctcccaatt ttgtgctagg cagagaaaag tagaaacaac cagatgatgt ccaccctgct 44520

agttctaaat cctcttttgg gtgcttggta aggagaggtg gagggaaggg aatgtgagga 44580

agggtcgttt agggaaataa atactggttg agcaatgggg cggtggctct agctgtggtc 44640

agcactgcac agagtagggg tctggaaagg agaaagaggc agaagaatct ggcttgaagt 44700

agatgcaaat acttacaacg aggaaacttt ctctgccaga ttcaaagggg cacatggcct 44760

ttgtaacctg gagaatgagg tgaggttaga gctgtgtttc ccttaccact ggcttatcag 44820

tgcctggcac agtacctaac acatagtagg cactcaataa atattgctca aagaatgttt 44880

cagaccatgc ttgtctgcag gagagctcat cttgcggagg ctgcatattt gctaaatgaa 44940

cgaataaatg attggtgaat gtttgtgggt agaagccaag aaagaggaaa actgacctct 45000

atggcatgac agaacgagta attttaggaa gtgacagggt ccaaggtgac caagtgatta 45060

tgtcctcagg agaaatggca gcaatgagac acctgtggtt gtcctcaacc tccaaaccat 45120

tctccacata cccatgacct gaccctaatc tcaaaaccca acctggcaag ctctggggaa 45180

ggtagtgctt agagacaatt tgagatgaag gttgaagaga gacaaagaat ctgggcccag 45240

ggagacaaag tcatttgggc ccagggagaa tgtggatttt ggggaaccaa taggtggaca 45300

tgggtcaaca gggagaagag tgcactgttc cattttttcc tggatatgac aatcaactgg 45360

aaatgatcag agggaggggt tgttgatgtt gaccaggcca ttgaacaaga ggcatgtgca 45420

caaattgaaa agtcagtcca ccttactctt tctgcgagtg ggaacgctta ggtccagagg 45480

gcaagagaac agaactggga tcacaggggc ctggagcagg tgtctcctgt ccagagcttt 45540

gaagttggtc cacttctaca aaagtatgtc tgtgtttggg agtgttactt attcttctgt 45600

ccctctgttc aaccaaaacc aaaaataaaa caaaacagat aaaaagaaaa caaaaccaaa 45660

aatccagagc ccacttacaa agggtttctc attagggcca agacaggttg ctaaagccaa 45720

accccatgtc cccagattcc agacaaaact cccctgctcc agtccacatc acctggaggc 45780

acccaccatg aaactgcact tggtgattgt ctcaaaaacc tacttgtact gcttatctca 45840

ggccatctgt gtttcccaaa ttcttgctat acaaacttgg ctcaatgtat aaaaattccc 45900

attgaaaata atgagaattt ggtatcaaag tgaagtccta gaaagaggtg ggagtgtggg 45960

gtggaggagg gacaggggaa caaaagaaaa atttcaaata gaaagtgcag agggatgggg 46020

aaaatgttga gtgaccaagc tagtccccga catccgtgtc tcggtcccct atgagccaga 46080

gaacatggaa gctgagtgct aggagcccag tgccaagcag gtcccccaga gcagtcaagt 46140

atgggatgga gaagttgtcc gggtccaggc cccggcccca catccagtgc accatccagt 46200

ctgcgatgta caggagaatc agcacctggg gagacaaaag ggcccagcct atagccatcc 46260

tctctctcca gggggagcca gaggccacat gatcagaccc attcagtgca tcacagggct 46320

tggctggcca taatccttac tttttaatct tcctctttcc accccagcaa cctctctgtg 46380

tttctccacc ccacaattac cactttgagc agacaacagg cacaataaga gctctttaga 46440

aaggaagatg ctgctacgac ccagggtcac ccacatcagc attatattct gctcccaagt 46500

ttaccaaaca aggagcttcc tgacctaaat cctacttgtt gcagaaactc agccctcttg 46560

cctctaactc tgggtcacct cattctgggg tcactccaat tcctgccatg ttggcctttc 46620

caggagcatg gcagggggct ggctgctgga gtcagctttt cctaaaggga tgtcttctct 46680

cggcagagtg gtataaggat agtgtttgaa gctgctgctg gcggttgtct taagtaacag 46740

atgatcatcc caagctggct tcctcctgac ccttttcatt ctgttgttgt ggcttttcat 46800

tccattcttt tccattgttt gggctcaaag aatgcaagtt tattttaaca ttagtcctaa 46860

taaagttgaa ttgtgaataa gtcacctcaa tattaatata aagcagacaa tgcataccaa 46920

ttcaaaatac atttgacatg atctgtggct ttgattttat ctgtgttctt gcttaccttc 46980

tattaataca gataattaag aatgactcag atcagaatct cgctagtggg cccaggacca 47040

gcagcatcaa caacagctgg gtgcttgtta gaaatgcaaa atctctggac ctcttgagtc 47100

agagtccgca tttaacaaga tttacatgcg cttttaagtt ggcgatgccc ttgatctaga 47160

ataccacggt ggggcaggcc tgaatctcct agaatctgca cagtgcctga catttgggct 47220

ccctaaatga ctgcatctgc gtgagaatga aacagattct gacggtcaag ctctaataca 47280

gtcagtcatc tgggaggttt tacccttgac tatactttgt aaatactgtg gtatgaaaat 47340

tctgccctaa gggatccaga tgcgggaacc acagccctta agggatccag aagccagaac 47400

tgatacctat ttttaggcaa tcttggttgc tctcctgtcc tctctctttt ttttttttta 47460

aattttaggc tctgcctgag agaatcctgc cctctctcca aggaagccag agatgactct 47520

tcacattctc ccttgctctg cacacagctg tcttgtgcta ccacggcctt ggctccccta 47580

ccttccataa cttcacaccc tttccagcag ctgcagaagg gtcccaaata aagagcatgg 47640

ccaggaggcc tttgggacat gccctaggaa gggaatggta gcagctgtcc tcacattcca 47700

gcttcttgag caggccccag gacacacagc tgggaggagg tggcaaaggg atcaataaga 47760

gcctttacag gggatcaagt gtcacgctct ggccctccac agatctagag aggacagcag 47820

aggcaagaat gtctccaggg tattcatcat gggtgatggc actgaggctc atcctttcca 47880

attcaggcca agggatgagc tgaaaacctc caccacagac ccctggcata tccaccccca 47940

tcttcccacc acgtgctgca ctggaaccca ctagcaatgg aatgtggcag cttggctaac 48000

tgctgcaggg caacagcata tggctgattc tcagccccgc ccctgccaag atgtccgaac 48060

acaacctttc taaagctggg ggtgaggggg ctggagggat aggtgttaat caatacagga 48120

ctggcgaaat aaaattatgg tctgtgtatc acagaatatg atggagctgt aagaaaatca 48180

gggtgttgtt ctctttatgt attgatatgg aatgatctca aagataaact taaatgaaaa 48240

atgcagggtg cagacagtgt gtacagtata ctacatgtgt gtaaaaaaga ggaaggaaat 48300

aagaaaaata tatgtttact tttataaata tagaataggt ttgaaggaca cacaagaaag 48360

ggataatact gatggtctgt gaaggagaga aacagatggc tgtgaagaag aggacagaaa 48420

agaagacttt tcactgaggt ttctctatga accttaaaac atttttgaac tatgtgacta 48480

ttcaaaaatc aaacctataa aaatttaaaa tgtaataata agggtggatc tagctgtaaa 48540

gggatagcat aagggagcct tgtggtgatg aaacagtttt ctgtattttg gttatagtgg 48600

tggtcacaca aatctccact tgtgataaaa actgcataga acgaacacac acgcacacac 48660

acacacgagt gcatgtaaaa ctggttcaat ctgaataagc actgcagatt gcatcaatgc 48720

taatttcctg attttgatac tgtcctaaag ttatgcaaaa tgttattttc ccatggaggg 48780

aaattgggta atggtatatg ggatttctga accttttttt tgagaacttc ttacaaatct 48840

gtaatgattt caaaataaaa agcttttaca atgtaataat acaagaaggg agaaagtgtc 48900

atggtattat ggaatggcaa ggctacacag gatccgacag attatctagt gcagtggttt 48960

tgacagtttt tcaagctatg ggatgcttta tgcaaatgaa tttcttgaga aagctcaaca 49020

tgcaaaacag agccatgtgg agctctgtgg ccagtggggt tggcatgcaa accccacagg 49080

ctcagcatcc tacaaccacc tgcatgaaaa ctaccacccc tggtccattc ctctcaattt 49140

acaactgtat cgctgaggat cagggtagta aaagaacagg gtcaaagtca cacaccatgt 49200

tggtgactct actggaacta ccatctgttc tcctgcaccc ttgtctggtg tttatcagct 49260

gccctgacac agagaaagag gaggttgagt gtctaagagg ccaagtctgg cctcctctcc 49320

catccctgca gggcatcctg gctccttccc atgcccctgc tcctgccact ttgtacctgg 49380

agcagtgcag ctgtcatata gaagatgatg aagatgagtg tgagggtggt gtgcccgccc 49440

tgcatacagc tgatggtgta gaggaacacc aggtgtcctg ggaccacgag gaggaagagg 49500

acccgggctg agcgagaatt cacatctgga attggggaaa agagggtgta aagaggaggg 49560

gaggagaaga cctggcccca gaaggccttt caaagtcagg agggacggca ccatacccca 49620

gggcaacaga cagatgaatg tctctacttc tgcatcctgt ggtctccaac ccctagggtg 49680

ctccaggaaa agacagccct cctgcctggg ctctgccctt cagaacagct ggcacagccc 49740

agcagagaag gagaacctgt ggatctgggg ccccagctgt ctcttaagtg aagctcactg 49800

acagtaggcc cactcccccc agggctggga ggctgggaat ctgccgttac cagggctgaa 49860

gaaggtggta caaggactgg gacagcggcg aggagcttgc tcagagttct ctccgggcat 49920

tccattcatg tgcaggaagg tggagatgcg gctggcctgc actgccacca gattgccccc 49980

aacacctgca gagacacata cgggcttaga cacagacaga ggtcagaggg aggagtggga 50040

acaaaatgat cttctcactc tttgtcttct atctatagag gcactgtcct ctgtcttaat 50100

ttagggtcta tgtgggcatc acccattcac cagtccccaa aagaagagta aggcactaag 50160

aaggaagaca aagacccctg cttccagtgc atcttccagc ctcccccatc ctcccctcta 50220

tggagatgca gcagttccat gttcccctgg caaagctgtg acagtgccgg aaaggatacg 50280

tgcagatctg cagactcaga gagcctgact tctccaccag tagagcatgg gcacctgcct 50340

tcctacacag ctgaggggat gaagccctgc cttgcctcac cgcctcacct cctctccaat 50400

gctctgagca gacccaggca tcctatggtc atatgctgat tctggtaacc agtttcaagg 50460

atcaggtttg agctccaaac tctggaatct ggtatctccg ccaggacctg tgctgatttc 50520

tctgttgccc tcacatccca acctgctccc tgctcttctc agccttgctc tgagctctgg 50580

gggactggtc tccacagact gcattaccca gtctcccttg ctggctgctt ccactgtttg 50640

ttcagtggga ggcaacagca ggcaaccaga gggcaggtgg agagagagat caggtatttc 50700

tttcccaatc cctccctgct tttcagcatt ggcttcccca tgttgggggc aaagacagag 50760

ctttcactgg actcctataa tgctattccc tcgttactat ggtttgaata tagtttgtcc 50820

ccaacaaaac tcatgttgtg gcttggtccc caaatgtggt ggtgggaggt ggtgccttta 50880

agagtgaata ggttattaag atggattaat gtctttcttg ggaaaggtat agttcccatg 50940

agagtgggtt gtcataaagc gaggtttgcc tctcttgttt tgcccatttt gcacatgccc 51000

agttttcctt ctgtttctcc atttggatgt ttggatgcag catgaggccc tcactagaag 51060

cagctgctca atcttgaact tcccagcttc cagaactgtg agctaaataa acttcttttc 51120

gttataaatt agccagtttc aagtattctg ttctagcaac agataatgga ctatgatact 51180

ccccttgacc ctttaggagt agaggcttcc cattgctgct aatctctcgg tgcctcaaca 51240

tcccttatct gtccttttac tctgctccca cctctgtaag cagtcccttc attaaggtct 51300

tgaaccatct gagctagatt ctcgtctgct ggaaccctga ctgatacaga aaccaacccc 51360

ttaagtcctc ttctcctgtc cctttccttc ccccagccct gccctctgat agctgatgcc 51420

cagacctcac cattaatcac aggcgtgaag acagccatcc cagcaaagtt ggggtctgag 51480

acagtcttgt ccaagatgag gcctcccaca ctatagagac ataaagacaa aatgacgcaa 51540

agaccactga agggttctgc cttagtctct gggatgagag gtccaggccc acctatcctt 51600

ggagatgaag ggaagcacca tggctctcag gagttcctca ccatccagtc cttgcctccc 51660

ttgacaggat taggtgctgc tcataacatt ctgactgggc acagtgactc cagaacaagc 51720

tggtggtact gggtcaggca accacctcct gtacctgccc tctaagttgg ccggaggagc 51780

agcctgcagg actatctgcc tctgaggacc tctgttgttc tgcccactca gcatttactt 51840

ccctttctgg tgatagcact gggctcctcc acagggaacc acctctctca agctcagtga 51900

ctgtggctca ggtgacaatg cctcaatgat agctaggctc caggtccatc caatcagagg 51960

attgcagccc caaggccacg ctgactggtt tggagataat catgtcacca gatgagaagc 52020

agtggaactc tattctggga ctttgtggaa ctgttggggg aagaatctct cttcccactg 52080

gaatttgtag gagtaagaaa agcataaacc tgggctgtag gccatgacag agaagagcag 52140

agctaagagt cacagagtac tggggccctg atcttaggta tgtcgctgaa ttcagctgtg 52200

cctttttggt tctccaggcc aatacattct gccttggaat gaaacccagt tcagatggtt 52260

tctgtcattt tcaacagaag gacatgtgac tgaaacacta atgaacacat ttctagggtc 52320

tgacccccac cccatctctc cagggtttaa aaagaagtct gtggaagggt tggagtgggg 52380

taggagtgga tactcagggc cccagcctac ctgctgatgg ccatggcaat gataacaggc 52440

tcccagcccg agtacaacac ctcccttgtg gctggacttc gtcgggccag caccacccag 52500

acaggcagca gggccacaaa gaaagcacac accagtgggt agatgtatcg ccagtgatct 52560

gagagggcag aggtcagaag gagataagca ctgtgtttcc ctaccctaag tttctccctg 52620

gctcagcctg aaaatgtccc aacagatcaa tagcaagact acactaaatg ccagtaaaca 52680

cattatccca tttgatcttc actggcagct caggaggtag gtgggacaag ccttttccat 52740

tttatggatg aggaaaccaa gggagagagc tagccaggca ctgagccaat tagggacaga 52800

gaccagagga cagagactct aggtctcttg attggagccc agaaaacacc atctcccacc 52860

cgcttcttca ttttgcaagc agatctaagc ctctggttcc tttcctttac cctttccacc 52920

tcatccctcc aagagaagta agaatctgca ggaccacaca ggaatatact tctgatactt 52980

ttcccctgcc cctgatgcct gcttgaattt gagatcagta cttagcttca aatctctaag 53040

atcaggaatt attacaacct gtgttcaaac caaatagttt aagaacacag aagaaactct 53100

tgcacatttg caaactacta ccatctctcc caaccccacc caggactcca agaagccaca 53160

ctcttggtgg ctcactcagt tccaggtaga gtccccagct gatgcctgag agcagcgcca 53220

aggtgatgag gtcgcccagg ctggcagcaa tgggtgtggc cacgttgtct gggttgatcc 53280

caatcttgcg agagccaatg atgactccaa tcatgatcat acctggtgag caagtgggag 53340

gaggggcagg cagggtgaga agagataaag gagtctcaac aaacaaaaag agagagtgga 53400

agtgccaggt gcagtgttct ctggggcggc actccttact cctctatgcc ctccctttct 53460

tacccagtac cagggaggca atgaaggctg tggccacgct gctagcacag agcaggaagg 53520

cgtgcggaat actgaagtgg ccatcaggga tccagccaaa gacgacggct gcgatggacg 53580

ccaggaagcc caccaccgtg gcctgcacct ggggacagga gtggtaactc agaaagccct 53640

gagagcagtg ggcttcctaa gctgaggtca taagcatctg ggcagacctc ttcctggctg 53700

gcagtagccc caggaggcag gctggactgc atggtccagt cctcccaggc tggaaggatg 53760

tctttattta tttaggttca caccagccac tagtactggg cagtgcactc tgcaggagcc 53820

cagaacatgt tactggctga atgagtgtgc ctctggggat gggagtgagg ttcaacagag 53880

ccggagactt ccacccagag tcaaggcctg ggcctcaagg gggaaggctc ccagtctgaa 53940

cctgtgtggt ctcatgggga gatgggacag cagtggcaca ggacatctca gcactcctga 54000

gtctgctcat attgccagga tctgctggga ctcaaaggca cgtgcaccac caagaactga 54060

gaaccagaat gggtgatgct gggccctcct catcttgggc gttttcctgg gtaacctagt 54120

tacctcctag agctactctg gggaagccca gctcagtggg atactggaga ttcactcagg 54180

agcctgctgg ggctgaccta agcctttcag agattcctga ctaaggggaa gcttagccca 54240

ccctctctgg tccctgcctt cttacctgga tgagggccat gttcccagtg atcatccgcc 54300

agagctcctt gggtgtgtcc atgtgtccaa tgttggcctg ggaaagggag ggcaaggggc 54360

tggagcttca ggctggaaaa ggcctgaagc tccatccatt aggctcctgc ctagatcatg 54420

aggcagtaca tgtggcctaa gactctgaga gcaggacctg acagtcccgg aaaggtgaag 54480

caggatcagg gaaactgagg tgaaagggaa ggagctaaag ctgggctcct gcagctgggt 54540

ggggggttgg aagggggtcc cctaggcagt cgcctctctt cctctcagga caccagctga 54600

gcagaggcaa attctatcct ttgccctgct tatctgggca gcccattcct gcatgggtca 54660

gaacaaccca gtccctgact ctgaagccag cctgcgatcc cagggaagga aaggtcagct 54720

gctggtgcag gactgaggct ccaagagtat ttgggaagga gtgggtggca tgggccagaa 54780

gtcgggtatg gcaagtcagg gtgggttaac ttggatgcca cttctgtctg tcaccttctc 54840

tagactcttg accctgcagg gagatccctg gcctcctgag ttttatcatc tcccacctcc 54900

agcccagggc cctgtatctg ttcaggcccg gaatcgtcac ggctcacaac tgtgggaggt 54960

aggaatgacg agcagctcct ctctgtctgt ttccttaaag aagagcaagc tgccatgggt 55020

aaagggaatg ggatggccgg ggggctgggg gtgctgggag ggcctggctc tgctcccgcc 55080

cagctgcaaa gcaggggctg gagatcagag gaggccccca tctgtgcggg tggaggaacc 55140

agcatgatgc ctgcctgtca aagctgcaca aacagtcacg aggtggttga gaggtagggg 55200

gtggggcagg agtccagggg tggctgggac atccagaaac caagcctggg aaccactttg 55260

ggcagggagc ccctgcccag gagcaggaca cccagcctgg gatctcccta cacctgccca 55320

gccaggccct cccttcccac cccagcccca cactccaggc ctgggcagtg gagaagggct 55380

cgtagccctc tcccagtctc tggctctgtg ctgcccagag tcctctctgt gccccactcc 55440

tcccagccag gatactcact gcagtggaaa gccttgatgc cagggtcatt tccaggttcc 55500

ctttgagccc cagcagcgca ggcactagga tgaagacctc tgtcaccttc tggaagactt 55560

cccagtgcta cgaggtgaaa acagaaccca ggaatttcag tgccccaaag ggcgggcatt 55620

gaggttcaac aggtagtctg agatcagggg ctcgaggagg aaatagaatc agcagggagt 55680

gagcacgcca gccacctttc ctccaagaac cttggaacca aaccaagccc atgattcttc 55740

aaacaacaca accccccttc ctgccaccca gaaccaggga gatggagagg gtttctgccg 55800

gttgggagac tctcagtctg gccctacctt ttctgttttc ctggaggtgg gtggggaagg 55860

gtaatgggga cccacacagc tgagctgccc tgggctgcct gacaccccag aaatcctttg 55920

caatcccccc tcaaattgca atccctggcc cctttgcctt cagtccagtt tctctgttgt 55980

ctcagctgtg aagagttggg ctccacaggg ctggggccga ggctggggag attagagagt 56040

ttgtttggtt tctatctcca aggctttggg gcctcagcca cagcaactct ccttctgaac 56100

aggctctgaa ttaagactgt ggcccctaaa cctctgaatg ctggcctgca agaggctttt 56160

tatttctctg tgcactgcct tacccccaca caccttcctc aatctctcag ctaggagaag 56220

caagtgtact cctgtaggaa gagccctgga ccgggagtca ggagacctga gttctagtcc 56280

caagacaatc aatcctggct ctcctaccaa tgtctttggt taacatctat taaggactga 56340

ttgtgtgcta ggctaagtgc tctccatgta ttttctcttt taatcatctt aacaacccag 56400

taaggtaggg gctattttta ttctcatttt gtgaatgagt aaactggggt tcagacagta 56460

ttccactagt tagtgctaag aggactgcag gaaccaggac tggctatttg aaagctactt 56520

tacttgcaat tgaaactgag gacttgcaaa aaggtttagg attgtgtctc cctccctggc 56580

cctaacaggg taggtggttt cttttatttg ttttaagaat ggaaagcctc caggtttgga 56640

gagggaggag gaggcaggag gccaggcgga tatatatggg ttggtctgcg tatcatggct 56700

gggaggccaa gcactatgtt tggcaagggc actggaccaa gagccttggg tctcctacag 56760

gagacagcag ggatggcccc ctgcttatct ccccagtaca cctcctcacg tagaccggct 56820

ggacacctta gacaagttac aagggcctca agctcttgaa aaatgttcaa ttcagggacc 56880

agatcagatg gtctttcaag gcaattccaa cacccacact ctgtcgttaa agatgagcag 56940

tggccgagcg cagcggctca cacctataat cccagcactt tgggaggccg aggcgggcag 57000

atcacctgag gccaggagtt tgagaccagc ctgaccaaca tggagaaacc ccgtctctac 57060

tgaaaataca aaaattagct ggtcgtggtg gcacatgctt gtaatcccaa gctactcggg 57120

aggctgaggc aggagaatcg cttgaaccca agaggtggag gttgcagtga gccaagatcg 57180

caccactgca ctccagcctg ggcaataaga gtgaaactct gtctcaaaaa taaaataaaa 57240

taaaataaaa taaaataaaa taaaataaaa taaaatagat aaaataaagt tgagcaatac 57300

tgtagattag taagagatca cctcacactc attaggatgg ttactattta aaaacccaga 57360

aaataatcag tgttagcaag gatgtagagt ttgggctggg catagtggct catgcctgta 57420

atcccagcac tttgggaggc caaggaaagt ggatcacttg aggtcaggag ttggagacca 57480

gcctggcaaa catagtgaaa ccctgtttct actaaaaata caaaaattag ccgggaatgg 57540

tggcgcacac ctgtaatccc agctactggg gaggctgagg cagaagaatt gcttgaactt 57600

gggagttgga ggttgcagtg agccgagaca gtgccactgc actccagcct gggtgacaga 57660

gcaagactcc gtctcaagaa aagaaaaaag catgtggaga ttggaaccct tgtgcactct 57720

tggtgggact ataaaatggt gcagctgcta tgaaaaacag tacggtggtt cctcaaaaaa 57780

ttaaaaataa aactaccaca tgatccagca atcccacttc tggatattta tccaaaagaa 57840

tagaaagcaa ggtcttgaag atatattaat atgcccatgt tcatagtagc actatatgta 57900

ataaccaaga ggtggaagca acccaaatgt ccatcaagga atgaatggat aaacaaaata 57960

tgcaaagtgt ggtacatata taccatggaa tatttctcag ccttaaaaag gaaggaaatc 58020

ctgtcatatg ccacaacatg gatgaacctt gaggacatta ctctagtcat aaaaaacact 58080

gtatgataaa acactcagat aaaaaacact tatctgaggt atccaaagta gtcaaattct 58140

tttttttttt tttttttttc ttgagacacg gtctcactct gtggcccagg ctggagtgca 58200

gtggtgcaat catggcttcg ggctccagcc atcctcccat ctcagtctct cgggtagttg 58260

ggactagagg catgcaccac cacacctggc taatttttgt attttttgta gagacagggg 58320

tttcactata ttgcccaggc tggtctcgaa ctcctgggct caaaggatcc tctcgcctcg 58380

acctcccaaa gtgtggggac tacaggtatg aggcaccgag cccagcctaa gtagtcaaat 58440

tcatagaggc ggaaagaaga atggtggaag caaggcgctg ggggagcagg gaaaagagtg 58500

agtgtttaat aggtacagag cttcagctgg ggaagatgag gagttctgga gatctgttgt 58560

acaatgaggt gaatatacgt agtactattg aacagcacac ttaaaaaagg gttaagatgg 58620

taaattttat gttacacatt ttttaccaca ataaccaaaa agataagcaa ggctatcaga 58680

gggggtgggg ctagggcttg agaccaggag aggctgggcg ctgggtggcc ctataaggca 58740

agaacctctc ttttcctgtc ggagctctcc ctgtgtgaat ggggtgggag gcctcacagg 58800

atcctgggga aggactcatt tcactggggc tggagcatac gcttgcctga gcttctggtc 58860

tggccaagac ctgggactct ccttagggaa gccccaccgt agggactaac aatggggaag 58920

agactgcccc ccatctcaac aacagttact gggcttccag gcagccaaag gatgagccag 58980

ccctctccaa acactgaagg gaagtgaatc ccttcccacc ctaagacccc ccagagagaa 59040

ggcagcccca gtgagcccca tctgccctgg agcaacttgg ggactgttac tcttggacac 59100

ggctggtaca taccactgag gctccagctc cctgcagtct ggccttgctc tgcttcgcct 59160

cctgcttcct cccaaaccag ctccagagag gggaaaaatc cccctcttct cccctccctt 59220

gcgggctttc aaagctcctc tcagagcaca gcctcagagc tcatcttcac cccaaaacac 59280

cagctccacc ctgcttccaa gctgccagct ccatgaaaat ctgacttcta caggtaggtg 59340

tatgtaatgt acatgtactc atagaaagaa aaagcccaca agaagactcc acatgtatgc 59400

aagcaatgtt ttctgagctg tttgtaatga atattgcttg cttttattat tatttaagaa 59460

aaatgtataa agctcagcta agttcctctg gttgctacca taagcacccc aaatctctct 59520

acctcccagc acaatgcacc ctctccttat agctacagga ggatctacaa agtacggagt 59580

gttaggagca gtgggataag agacagacag gcaaggagag ggagcagtca cagtaacagc 59640

ccctatgcaa aagtcttccc gactcttaca gaatatagcc tggaacctca aacgacctga 59700

ccccaggcca gcaaagcccc gagttggcac ttgggtcaga gaagccaatt cctacacaca 59760

tactttcttc acccacaaac cctctcaaag ctgcagctca ggcagaaagc ctctgacaca 59820

tgggcatgca taggactctg gaaagagatg ctggaggctg gagatgtgga gccagcccag 59880

gaatcaaggt ggccttaagg ttaggagtag acaggatggg caggggcaga cagtgagtag 59940

ctcttcttcc tcctttggcc aaattcagcc ccatcctgtt catccaggct aggagtatct 60000

gcccctgaag tcacacccgt ctgccaagca gccacccacg ccttcctgct ctgatgggga 60060

tgaaaaaggg catgttccag gaagtgctgc cctgcttgga ggtgagggga gcatctagat 60120

tttaactact tctcagaaat gcagctcctc atctctttcc ttctctaacc ttggtgtgag 60180

gggctcatgg atccctggta ttctgcaggt gagggaatag actgcctccc ccatagccaa 60240

aatgaatcct gaggcacagc aatcacctta agtctcgatc ctcagtggtc ctgccagtac 60300

ctcttccttg cccttccaat gcgcctcttc catccttcac ctactccaat ccaagctcat 60360

acactgaccc tgacctgcct tcaagggttc tggcctgagc cacctcagga gggtagcagc 60420

agggaagaaa gtcagagcca ggaatcaaga cattaagaag acttaaattc ccagatacaa 60480

gctttaggct ccaggctggg gagaatctgg gctcctgtgg gagaagctgg gctggagctg 60540

atcttttagg gtaaggactg ggtcagatga gattacagta accccaggct tcacctgtct 60600

gaggcttttc caaaaggcat cctctaggtg tccaggaaag ggctttaggg caacacctgg 60660

atatacggtg caggacaaag caggagcaaa gcaaatgggt ccctgacagc aggcagggga 60720

cagagatggg aaaagcagaa ggggtggtca agtgaagcag ataagagtgt cccgcccagg 60780

gatgctgcgg ccacaggagc aggccaggca caggaggaat acttccttcc cactctactt 60840

ccccaaggcc acgcttgcca tgcactttca tcgttgtaga cttctgtata cacagcagga 60900

tctactttgt tcaagatcct tagcaggtga gtagtgagtt aaaataagga cctgtcttaa 60960

cctagctggg aattaaagtg cagatgggca cactacttcc tgtcacccca tccctcacaa 61020

aggctgacag ataaagggag tgagacatcc ggagcagcct aggaccaaac tgggcagatg 61080

ggcacaaggc tcctgcagtg aggaggctcc catatcccaa cccccttcag aagccaacac 61140

caggccccag cctgcttccc ttccagatgc tgcaggaact ttcagccatg gttgggaagt 61200

tagagcagat ggaggtccat ttatcctttt cctactttgg gccagcacca gccccttatt 61260

tcactgcaga tgctgctgtg cacatttacg ggagggcaga taagaggagg cagaaatgac 61320

ccttctagga agagagtggg ttttcttttt cttttaaatt aaaattaaaa ttcaactttt 61380

tatttttatt ttttttgcat ctcgttggtg aaaaagaaac caagttttct acagcagcaa 61440

ggaaagatgg gggccctgga ccaaggagac atctgaacca ggcccaatat ggactcctgc 61500

tcagttgctc caagatgctg gggacactcc atagccagct tctccagatg gccccatggt 61560

cttggggcac tttcagattc tcttactgaa gtccatctta gcttatgaag gggtaatata 61620

ggtgtgaatc cttagaaacc atcccgctct tcctgaatct gagattgtca gtcccccttt 61680

tactcccatg gatgtggaat aacaatctcc ctgtggtccc atgtctcggt ccctctgcac 61740

tcaccccctc ctgccatctc actgttgcca tctgcagggc ctgctccagc ccagctgtag 61800

caggaaggct gggggaggag gacatggaca aacctgtcat tgtcatccac accaagtgga 61860

ccctagctct gaggtgagct gaggtgccac actgagagca gctccaaagg ttccctaccc 61920

aggcctctca atgtctccag ctcatgggcc tggagtgggg atggggcaac acatcagaaa 61980

ctttggaatc taagtgtgtg ggatgggtag gggtgtggtg tgtatgcggg ggtccctccc 62040

taccccatct aagaactttt attcttgctc acttcagaag ataacgctaa ttaatccaga 62100

ttctaatcca cccagttctt caaggaaaga caaagaaaat aggctcctcg tagagtcttt 62160

tttttttttt tttttttttt gagacagcgt ttggctctgt tgctgaggct ggagtgcagt 62220

ggtacaatca tagctcattg cgacttcaaa ctcctgggct caagtgatca ctcctatctc 62280

agcctctcaa agtgctggaa ttatacgaga atgccaccat gcctgactat tttttttttt 62340

tttggtagag acaaggtctc actttgttgc ccaggctggt cttgaactcc tggcttcaag 62400

cgatcctcct gcctcagcct ccaaaagtgc tgggattaca ggcataagcc accatgccca 62460

gccagagttt tttttttttt gagttggagt ctcgctctgt cacccaggct ggagagcagt 62520

ggcacgatct tggctcattg cagcctccac ttctcgggct caaatgattc tcttgcctca 62580

gcttccaagt agctgggact acaggtgcct gccaccatgc ccagctaatt ttttgtattt 62640

ttagtagaga cggggtttca ctgtgttggc cagactggtc tcaaactcct gacctcaggt 62700

gatccgcccg tcttgacctc ccaaagtgct aggattacag gcatgagcca ctgagctcgg 62760

ccccctctcc gagtttttaa ccaactggtg aaccatggcc atggcgaagg ccacaggggt 62820

tagtggtgtg ggctctaggg tcagatgacc agggacaagt tacctaatcc ctctaagcta 62880

caagttccct acctacaaaa tggagatgat tatggatcct acctcatggg cctaccgaag 62940

agccccccaa atgcttcccc actaccatta ctattatcca gaaaataaga caactctctt 63000

cagttcacct ctggaccctg gctctgcaac cctttgtagg tatcaaacca gaatgcaaca 63060

acttcaagaa tgagaactga ggccacagga aaggaataga agaggtgaag aaaaatgtta 63120

ccctagtctc tttctcctcc tcctactgtc ccagtcccgg ccctaggggg aggagcagcc 63180

cctagcagga ggaacccaaa gctccaggct ggcttcccaa cctccatgta ggaacaacct 63240

tcaagataac agttttgggg aaagggaggg cagtgcaagg cactttggca tgacttaaac 63300

agcactctca tgagcaacca aaggtgcagg gttcccctga agggctcaag tcaaagttct 63360

ccatgtgcac acactatcaa gaaccaaccc ccttcagtct cccaggaca 63409